# EUROPEAN PATENT SPECIFICATION

(11) **EP 4 279 909 B1**
(45) Date of publication and mention of the grant of the patent: **18.11.2026**
(21) Application number: 23194111.3
(22) Date of filing: 03.07.2019
(51) Int. Cl.: A61B 5/00, A61B 5/20, A61F 13/42, G01N 27/12

(54) **SUBSTRATE, ABSORBENT ARTICLE FOR WETNESS MONITORING**
SUBSTRAT, SAUGFÄHIGER ARTIKEL ZUR NÄSSEÜBERWACHUNG
SUBSTRAT, ARTICLE ABSORBANT POUR LA SURVEILLANCE DE L'HUMIDITÉ

(43) Date of publication of application: 22.11.2023
(62) Document number(s) of the earlier application(s) in accordance with Art. 76 EPC: 19184167.5 / 3 760 104
(73) Proprietor: Ontex BV, 9255 Buggenhout (BE); Ontex Group NV, 9320 Erembodegem (BE)
(72) Inventor: HEIRMAN, Lisa, 9250 Waasmunster (BE); HELLMOLD, Jens, 59269 Beckum (DE)
(74) Representative: Cobbaert, Dries

(56) References cited:
- EP-A1- 3 415 130
- WO-A1-2013/097899
- WO-A1-2018/229017
- US-A1- 2009 005 748
- US-A1- 2010 241 094

## Description

### TECHNICAL FIELD

The invention pertains to the technical field of absorbent hygiene products. In particular, the present invention relates to an absorbent article for absorbing body fluids and exudates, such as urine and fecal material. More specifically, the present invention relates to absorbent garments, such as disposable diapers or pants for example for babies or adults, which are configured to collect and contain fecal material and avoid leakage.

Most particularly the invention pertains to diapers or pants, components thereof, and process of making, comprising an exudate detection system capable of automatically monitoring the distribution of exudates within the diaper or pant warn by a subject, and automatically providing a warning to a care giver when the risk of leakage is elevated and said diaper or pant should be replaced.

### BACKGROUND OF THE INVENTION

Disposable diapers conventionally include a chassis having a liquid permeable topsheet, a liquid impermeable backsheet and an absorbent structure sandwiched between the topsheet and backsheet. The chassis has a front body panel which, in use, extends over the stomach and front hip area of the user, and a rear body panel which, in use, extends over the back and the rear hip area of the user. Each of the body panels has a waist portion such that, when the diaper is fastened around the waist of the user, the waist portions provide a continuous encirclement of the user. In order to fasten the diaper around the waist of a user, a fastening system comprising fastening tabs is commonly employed. Fastening tabs may be provided on side panels which extend from lateral side edges of the diaper chassis.

Disposable pants have a similar construction but typically comprise front and back elasticized belts at either end of the absorbent structure and are sealed together at lateral side seams to form an underwear-resembling product that can be worn by a subject by pulling it up over the legs and may be removed either by pulling it down in the opposite direction or by tearing the side seams.

Inherent with the use of such absorbent articles, eventually one or more wetness and/or exudate events will occur and thus a need for change of the absorbent article arises. Particularly in home care and/or institutions, handling elderly with incontinence problems, knowing when to change the diaper/pant of a patient is of the utmost importance. Indeed, changing the diapers too early results in unnecessary cost and waste and changing too late results in further cleaning costs and time, and uncomfort or discomfort to the patient. This problem is exacerbated by motion, indeed taking into account that a subject wearing the article may move into different positions the saturation thereof and/or risk of leakage may vary considerably based on the position and time the subject remains in that position during and/or after one or more wetness/exudate events.

Absorbent articles possessing different types of detecting means are known, and help to alert a user or caregiver to a change within the article (e.g. a soiling event). Such detecting means allow the user or caregiver to readily determine whether or not an absorbent article needs to be changed, without the need for close inspection or removal of the article.

Commonly known detecting means which can be incorporated into absorbent articles are chemical substances which alter their form or nature upon contact with liquid. For example, an indication that an absorbent article is soiled can arise from colour changes or the appearance or disappearance of an element on the absorbent article. Such detecting means are useful in certain situations, but less so in institutions such as child-care centres, care centres for the elderly or hospitals where the status of a large number of wearers must be monitored, often by a limited staff. Determining whether the absorbent article is soiled or not still requires the wearer to be disturbed, as the coloured element must still be visible to the caregiver. This often requires that the wearer be moved, and their clothes removed or adjusted.

EP 3 415 130 A1 refers to an absorbent article, components and process therein using detecting means that provides the advantages of electrical detection, yet which is cost-effective, simple to manufacture and readily disposable. Nevertheless, the solution implemented in EP 3 415 130 A1 does not allow to identify the exact zones or locations at which the absorbent article is wet. WO2018229017 discloses a further substrate for incorporation in an absorption assembly for automatic detection of wetness.

There is thus a desire for absorbent articles comprising detecting means that provide accurate and reliable sensing of the different wet zones of the article enabling saturation monitoring (expression of the saturation degree of the absorbent core of the absorbent article) over the full length and the full width of the absorbent article, and that can be standardized across a range of products and sizes in order to limit production cost and thus the end cost to the users and institutions.

There is further a desire for detections means that alternatively or in combination provide on-time alerts based on the risk of exudate leakage from the absorbent article, without the need of simultaneously monitoring the position that the subject is in during and/or after one or more repeated wetness/exudate events, which result in reduced operation costs since the interpretation of the received signal into the current diaper status only requires limited intelligence.

Accordingly, it is the object of this invention to create a substrate, an absorbent article and a method for making such an absorbent article.

### SUMMARY OF THE INVENTION

In view of the above-mentioned problems and disadvantages, the object of the present invention is achieved by the solution provided in the enclosed independent claims. Advantageous implementations of the present invention are further defined in the dependent claims.

According to a first aspect of the invention, a substrate suitable for incorporation into an absorbent article for monitoring and detecting the presence of wetness therein and/or risk of exudate leakage therefrom, is provided. Said substrate comprises a conductive pattern disposed on a first surface capable of being arranged proximal to a body facing side of the absorbent article, wherein said conductive pattern can be brought in electrical communication with a clip-on data processing module, said conductive pattern comprising: a plurality of connection tracks; and a plurality of sensing tracks connected to said connection tracks, and wherein the conductive pattern is configured in a manner that addressing multiple combinations of said plurality of connection tracks with corresponding terminals of the clip-on data processing module result in multiple electrical circuit configurations for measuring resistance, impedance and/or capacitance therethrough. The plurality of circuits defined by the conductive pattern comprise circuits with an open configuration, and wherein the conductive pattern extends symmetrically in a direction substantially parallel to the length (L) of the substrate and a direction substantially perpendicular thereto, defining a plurality of independent electrical circuits, and wherein combinations of said electrical circuits are in electrical communication by selectively addressing at least two of the connection tracks with at least two of the corresponding terminals of the clip-on data processing module, and wherein resistance, impedance and/or capacitance measured through said plurality of circuits determines the wetness through corresponding sensing zones that are further directly related to body position of the wearer. Advantageously, accurate and automatic detection and monitoring of liquids and/or exudates in an efficient manner is enabled. In addition, said substrate is particularly suitable for being scalable for in-line mass production at high speeds. Further advantageously, this ensures that a reliable detection in several zones along the length and width of the final product will be monitored, enhancing efficiency and accuracy. Further advantageously, a direct relation of the monitored zones can be related to body position of the subject wearing the product without requiring using additional motion sensors and/or gyroscopes, increasing efficiency and saving time.

According to a first preferred implementation form of the first aspect, the substrate comprises a second surface opposite said first surface and capable of being arranged proximal to a garment facing side of said absorbent article. In addition to this or as an alternative, said clip-on data processing module is in electrical communication with the conductive pattern when connected at a position proximal to a first end of the substrate such to form at least one closed electrical circuit. This is beneficial, since the implementation of the inventive substrate into the final product is compatible with current production lines, which reduces operation costs compared to other solutions that require additional more complicated process steps and, furthermore, enables the use of the final product in a harmless manner for a wearer.

According to a second preferred implementation form of the first aspect, the conductive pattern 101 comprises an electrically conductive material, and is preferably a printed conductive pattern, the printed conductive pattern comprises, preferably consists of, a carbon-based ink and/or a conductive polymer-based ink, preferably the carbon-based ink comprising a conductive compound selected from the group consisting of graphene, graphite, nano-carbon-tubes and mixtures thereof, preferably the conductive polymer-based ink comprising a conductive compound selected from the group consisting of polyacetylene, polypyrrole, polyaniline and copolymers thereof, more preferably selected from the group consisting of poly(pyrrole)s (PPY), polyanilines (PANI), poly(thiophene)s (PT), poly(p-phenylene sulfide) (PPS), poly(p-phenylene) (PPP), Poly(acetylene)s (PAC), Poly(p-phenylene vinylene) (PPV), poly(3,4-ethylenedioxythiophene) (PEDOT), and mixtures thereof, most preferred conductive polymer-based ink comprising poly(3,4-ethylenedioxythiophene) polystyrene sulfonate (PEDOT:PSS). Advantageously, fast production is achieved whilst ensuring good compatibility with the final product.

According to a further preferred implementation form of the first aspect, said connection tracks are configured to allow an electrical current to flow from corresponding terminals of the clip-on data processing module when connected to it. Additionally or alternatively, said connection tracks extend substantially parallel to a length L of the substrate and substantially parallel to a longitudinal axis (y-y) crossing a first end and a second end of the substrate. Moreover, said sensing tracks are configured to undergo changes in presence of certain substances. Advantageously, the reliable sensing of wetness along the final product is ensured whilst providing continued and reliable wetness detection and monitoring.

According to a further preferred implementation form of the first aspect, the substrate further comprises at least one insulating layer preferably being liquid impermeable, adhered thereto and sized to cover at least a portion of the conductive pattern, said at least one insulating layer adapted to provide a seal and/or barrier to liquid and/or exudates from coming into contact with said portion of the conductive pattern, preferably wherein the sensing tracks remain exposed to liquids and/or exudates and are not covered by said at least one insulating layer. This is beneficial since an insulating layer, being an inherent component of the final product, contributes to define specific zones where wetness can be selectively detected, enhancing efficiency.

According to a further preferred implementation form of the first aspect, said substrate comprises one or more slits, and a pocket is formed between the first surface and the at least one insulating layer proximal to the first end, said pocket being in fluid communication only with said slit(s) and arranged to retain at least a portion of the clip-on data processing module therein. Advantageously, this arrangement enables a secure electrical connection between the clip-on module and the connection tracks in enabled whilst ensuring also its protection from soiling by internal and/or external elements like. Furthermore, comfort for the wearer is also enhanced.

According to a further preferred implementation form of the first aspect, the plurality of circuits defined by the conductive pattern comprise circuits with a closed configuration. In addition to this or as an alternative, at least one of the plurality of circuits defined by the conductive pattern is a circuit with a closed configuration. Additionally or alternatively, the plurality of circuits defined by the conductive pattern have a configuration that enables space for applying glue lines when incorporated into an absorbent article. This is beneficial, since the electrical properties of different circuits are used to enhance sensitivity on detecting and monitoring wetness. Further advantageously, appropriate electrical connection is ensured.

According to a further preferred implementation form of the first aspect, a thickness and/or a length and/or a width and/or a specific conductivity of at least a portion of at least one of the plurality of circuits defined by the conductive pattern comprises identifiers of the absorbent article that are detected when connected to the clip-on data processing module. Advantageously, this increases efficiency and reduces fabrication costs.

According to a second aspect of the invention, an absorbent article, preferably a disposable diaper, pad or pant, suitable for monitoring and detecting the presence of wetness therein and/or risk of exudate leakage therefrom, is provided. Said absorbent article comprising: a liquid impermeable backsheet; a liquid permeable topsheet; an absorbent core interposed between said backsheet and topsheet, wherein said backsheet comprises a substrate, the substrate comprising a conductive pattern disposed on a first surface and can be brought in electrical communication with a clip-on data processing module, said conductive pattern comprising: a plurality of connection tracks; and a plurality of sensing tracks connected to said connection tracks, and wherein the conductive pattern is configured in a manner that addressing multiple combinations of said plurality of connection tracks with corresponding terminals of the clip-on data processing module result in multiple electrical circuit configurations for measuring resistance, impedance and/or capacitance therethrough. Advantageously, accurate and automatic detection and monitoring of liquids and/or exudates in an efficient manner is enabled, ensuring scalable in in-line mass production at high speeds.

According to a first preferred implementation form of the second aspect, said absorbent article further comprises a removable clip-on data processing module having a plurality of exposed electrically conductive terminals capable of selectively addressing a plurality of connection tracks of a conductive pattern, preferably wherein the number of said terminals is at least the same as the number of said connection tracks. This is beneficial since appropriate electrical connection of said clip-on module with the connection tracks is ensured, since the clip-on module remains secured to the absorbent article whilst being protected from accidental disengagement during movements of the subject/wearer.

According to a second preferred implementation form of the second aspect, selectively addressing multiple combinations of the plurality of said connection tracks with corresponding terminals of the clip-on data processing module result in multiple electrical circuit configurations for measuring resistance, impedance and/or capacitance therethrough. In addition to this or as an alternative, at least a portion of the clip-on data processing module is retained within a pocket, said pocket positioned over at least a portion of the absorbent core with the core being interposed between said pocket and the skin of a subject when the absorbent article is worn such that when the clip-on data processing module is inserted into the pocket, the absorbent core provides a cushioning layer between said clip-on data processing module and said skin of the subject. Advantageously, this arrangement allows to selectively define various zones of the final product where it is desired to detect whether wetness due to liquids and/or exudates occur.

According to a further preferred implementation form of the second aspect, measuring resistance, impedance and/or capacitance of multiple circuit configurations defined by the conductive pattern selectively in contact with corresponding terminals of the clip-on data processing module enables monitoring wetness at different zones of the core of the absorbent article and/or monitoring a distribution of wetness in dependence of the actual and/or previous body position of a wearer of said absorbent article. Advantageously, this enables to effectively detect wetness and to generate a mapping of wetness with high accuracy, from which also body position of the wearer is directly inferred.

According to a third aspect, the invention relates to a method for making an absorbent article comprising the steps of: providing a liquid impermeable backsheet and applying a conductive pattern to a first surface thereof, the conductive pattern comprising a plurality of connection tracks and a plurality of sensing tracks connected to said connection tracks, wherein the conductive pattern is configured in a manner that addressing multiple combinations of said plurality of connection tracks with corresponding terminals of the clip-on data processing module result in multiple electrical circuit configurations for measuring resistance, impedance and/or capacitance therethrough; providing and adhering at least one insulating layer to said backsheet, said insulating layer being sized and positioned to cover at least a portion of the connection tracks of the conductive pattern to provide a laminated substrate; providing an absorbent core comprising absorbent material; providing a liquid permeable topsheet; and sandwiching the absorbent core between said laminated substrate and said topsheet. Advantageously, accurate and automatic detection and monitoring of liquids and/or exudates in an efficient manner is enabled, ensuring scalable in in-line mass production at high speeds.

According to a first preferred implementation form of the third aspect, the method further comprises the step of providing a slit and/or pocket on the backsheet enabling an electrically conductive portion of a clip-on data processing module to selectively come in electrical communication with the connection tracks of the conductive pattern. This is beneficial since proper electrical connection is achieved, increasing efficiency and accuracy in wetness detection.

According to a further preferred implementation form of the third aspect, the conductive pattern is in the form of an electrically conductive ink and the application step comprises the printing thereof onto the backsheet. Additionally or alternatively, at least one insulating layer is formed to have a width w, taken along an axis perpendicular to the longitudinal axis (y-y), being less than a width W of the backsheet. In addition to this or as an alternative, monitoring and detecting the presence of wetness at different zones of the core of the absorbent article is enabled by measuring resistance, impedance and/or capacitance of multiple circuit configurations defined by the conductive pattern and selectively addressed by corresponding terminals of the clip-on data processing module. This is beneficial, since a secured and protected connection is achieved in a simple and cost-effective manner, resulting in an optimum design that can be easily implemented in fast in-line fabrication processes.

### BRIEF DESCRIPTION OF THE DRAWINGS

Illustrative embodiments of the present invention are further explained by way of example only, and not for limitation, with respect to the accompanying drawings in which like reference numerals refer to similar elements. It is mentioned that the various features are not necessarily drawn to scale. In the drawings:
- Fig. 1A: shows a schematic top view of a substrate according to an embodiment of the present invention;
- Fig. 1B: shows a schematic top view of a substrate including the insulating layer, according to an embodiment of the present invention;
- Fig. 1C: shows a schematic top view of a substrate according to an embodiment of the present invention;
- Fig. 2A-C: show portions of the connection tracks according to an embodiment of the present invention;
- Fig. 3A-F: illustrate the operation principle of multiple usage of terminals, according to an embodiment of the present invention;
- Fig. 4A-B: show two exemplary electrical circuits for selectively connecting multiple terminals to multiple connection tracks, according to an embodiment of the present invention;
- Fig. 5A-C: show exemplary sensing zones defined by selectively connecting multiple terminals to corresponding connection tracks, according to an embodiment of the present invention;
- Fig. 6A-F: illustrate possible circuits resulting from connecting the same pair of available terminals;
- Fig. 7A: shows examples of closed circuit configurations defined by the conductive pattern, according to an embodiment of the invention;
- Fig. 7B: illustrates an exemplary closed circuit configuration connected to corresponding terminals and to a voltage source;
- Fig. 7C: shows examples of open circuit configurations defined by the conductive pattern, according to an embodiment of the invention;
- Fig. 7D: illustrates an exemplary open circuit configuration connected to corresponding terminals and to a voltage source;
- Fig. 8A-B: illustrate the electrical characteristics of exemplary open and closed circuit configurations, according to an embodiment of the present invention;
- Fig. 9A: illustrates an exemplary conductive pattern according to an embodiment of the present invention;
- Fig. 9B: shows exemplary ends of sensing tracks according to an embodiment of the present invention;
- Fig. 10: illustrates further exemplary conductive patterns according to embodiments of the present invention;
- Fig. 11: illustrates the electrical principle allowing for product identification, according to an embodiment of the present invention;
- Fig. 12A-E: illustrate open circuit configurations and its specific response to the presence of wetness, according embodiments of the present invention;
- Fig. 13A-D: illustrate open circuit configurations and its specific response to the presence of wetness with and without the insulating layer, according embodiments of the present invention;
- Fig. 14A-C: illustrate an exemplary embodiment of a clip-on data processing module as described in embodiments of the present invention;
- Fig. 15: illustrates a schematic top view and a schematic lateral view of an absorbent article, according to a preferred embodiment of the second aspect of the invention;
- Fig. 16A-D: illustrate circuits and sensing zones within an absorbent article selectively activated by combinations of multiple terminals and corresponding multiple connection tracks in an exemplary embodiment, according to the second aspect of the invention;
- Fig. 17A-D: illustrate further circuits and sensing zones within an absorbent article selectively activated by combinations of multiple terminals and corresponding multiple connection tracks in an exemplary embodiment according to the second aspect of the invention;
- Fig. 18: shows an exemplary mapping of wetness along an absorbent article, as described in embodiments of the present invention;
- Fig. 19: illustrates a schematic top view and a schematic lateral view of an absorbent article, according to an embodiment of the second aspect of the invention;

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment. "About" as used herein referring to a measurable value such as a parameter, an amount, a temporal duration, and the like, is meant to encompass variations of +/-20% or less, preferably +/-10% or less, more preferably +/-5% or less, even more preferably or less, and still more preferably +/-0.1 % or less of and from the specified value, in so far such variations are appropriate to perform in the disclosed invention. However, it is to be understood that the value to which the modifier "about" refers is itself also specifically disclosed. "Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein. The expression "% by weight" (weight percent), here and throughout the description unless otherwise defined, refers to the relative weight of the respective component based on the overall weight of the formulation or element referred to.

The recitation of numerical ranges by endpoints includes all numbers and fractions subsumed within that range, as well as the recited endpoints, except where otherwise explicitly stated by disclaimer and the like.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants, diaper holders and liners, sanitary napkins and the like, as well as surgical bandages and sponges. Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. Disposable absorbent articles can include a liquid pervious top sheet, a back sheet joined to the top sheet, and an absorbent core positioned and held between the top sheet and the back sheet. The topsheet is operatively permeable to the liquids that are intended to be held or stored by the absorbent article, and the back sheet may or may not be substantially impervious or otherwise operatively impermeable to the intended liquids.

The absorbent article may also include other components, such as liquid wicking layers, liquid intake layers, liquid distribution layers, transfer layers, barrier layers, wrapping layers and the like, as well as combinations thereof. Disposable absorbent articles and the components thereof can operate to provide a body-facing surface and a garment-facing surface. Preferably, a diaper comprises a liquid permeable "top sheet", a liquid impermeable "back sheet", and an "absorbent medium or core" disposed between the top sheet and the back sheet. The top sheet, back sheet and the absorbent medium could be made from any suitable material known to the person skilled in the art. The top sheet is generally located at or near the bodyside surface of the article, while the back sheet is generally located at or near the garment-side surface of the article. Optionally, the article may comprise one or more separate layers which are in addition to the back sheet and are interposed between the back sheet and the absorbent medium. Top sheet and back sheet are connected or otherwise associated together in an operable manner.

The "absorbent medium" or "absorbent core" or "absorbent body" is the absorbent structure disposed between the top sheet and the back sheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, l-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material.

"Registered" (e.g. Registered design) refers to, for example, a print design that varies along the length of a continuous web/film (and is repeated at intervals throughout) and that hence requires some form of recognition on where a processing action must happen, e.g. a cut, to ensure that consecutive discrete products formed from the continuous web/film each have the same print design.

"Acquisition and distribution layer", "ADL" or "surge management portion" refers to a sub- layer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the top sheet and the retention portion.

The term "adhesive" as used herein is intended to refer to any suitable hot melt, water or solvent borne adhesive that can be applied to a surface of a film layer in the required pattern or network of adhesive areas to form the film-nonwoven laminate of the present invention. Accordingly, suitable adhesives include conventional hot melt adhesives, pressure-sensitive adhesives and reactive adhesives (i.e., polyurethane). As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used herein, the term "associated or joined or adhered" encompasses, unless expressly stated, configurations in which e.g. a top sheet is directly joined to a back sheet by affixing the top sheet directly to the back sheet, and also configurations wherein the top sheet is joined to the back sheet by affixing the top sheet to intermediate members which in turn are affixed to the back sheet (i.e. indirectly joining). Top sheet and back sheet can be affixed directly to each other by attachment means such as an adhesive, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix top sheet to back sheet. It should be readily appreciated that the above-described attachment means may also be employed to interconnect and assemble together the various other component parts of the article described herein.

The terms "back section" and "rear back section" are used herein as synonyms and refer to the area of the absorbent article which is contact with the back of the wearer when the absorbent article is worn. The term "backsheet" refers to a material forming a liquid impermeable cover of the absorbent article. The back sheet prevents the exudates contained in the absorbent structure from wetting articles such as bedsheets and overgarments which contact the disposable absorbent article. The back sheet may be a unitary layer of material or may be a composite layer composed of multiple components assembled side-by-side or laminated. The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material.

The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials. The terms "belly section" and "front belly section" are used herein as synonyms and refer to the area of the absorbent article which is contact with the belly of the wearer when the absorbent article is worn.

As used herein, the "body-facing" or "bodyside" surface means that surface of the article or component which is intended to be disposed toward or placed adjacent to the body of the wearer during ordinary use, while the "outward", "outward-facing" or "garment-side" surface is on the opposite side, and is intended to be disposed to face away from the wearer's body during ordinary use. Such outward surface may be arranged to face toward or placed adjacent to the wearer's undergarments when the absorbent article is worn. "Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "breathable" refers to layers, preferably films or elastic laminates, having a water vapor transmission rate (WVTR) of at least 300 grams/m2 - 24 hours.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

Further, an absorbent article can comprise "containment flaps" or "barrier cuffs". The containment flaps are generally thought to be particularly well suited for the containment of fecal matter and to prevent the lateral flow of liquid waste until such time as the liquid waste can be absorbed by the absorbent article. Such containment flaps generally comprise a proximal edge, intended to be attached to the absorbent article, and an opposite distal edge which is generally not attached to the absorbent article along at least a portion of its length. An elastic member is generally located adjacent the distal edge to assist in maintaining the containment flap in an upright condition and in maintaining a sealing relationship between the distal edge of the containment flap and the body of a wearer during use. The elastic member is generally located between two layers of material so that the elastic does not come into contact with the body of a wearer. The containment flaps may be manufactured from a wide variety of materials such as polypropylene, polyester, rayon, nylon, foams, plastic films, formed films, and elastic foams. A number of manufacturing techniques may be used to manufacture the containment flaps. For example, the containment flaps may be woven, non-woven, spunbonded, carded, cast, blown or the like.

An absorbent article can comprise leg containment gaskets. Leg "containment gaskets" help prevent leakage of bodily exudates when the wearer exerts compressive forces on the absorbent article. In particular, the stiffness of the leg containment gaskets prevents twisting and bunching of the leg openings of the absorbent article which can lead to leaks. In addition, the elasticity and conformability of the leg containment gaskets ensures that the bodyfacing surface of the leg containment gaskets provides an adequate seal against the body of the wearer. The physical properties of the leg containment gaskets, such as the thickness and stiffness, also function to space the bodyside liner, outer cover and absorbent core away from the wearer's body when in use. As such, void volume is created between the wearer's body and the bodyside liner and absorbent core of the absorbent article to help contain body exudates. "Mechanical bond" is an attachment between two or more elements, components, regions, or webs and may comprise heat bonds, pressure bonds, ultrasonic bonds, dynamic mechanical bonds, or any other suitable non-adhesive attachment means or combinations of these attachment means.

"Hotmelt adhesive" means a formulation that generally comprises several components. These components typically include one or more polymers to provide cohesive strength (e.g., aliphatic polyolefins such as poly (ethylene-co-propylene) copolymer; ethylene vinyl acetate copolymers; styrene-butadiene or styrene- isoprene block copolymers; etc.); a resin or analogous material (sometimes called a tackifier) to provide adhesive strength (e.g., hydrocarbons distilled from petroleum distillates; rosins and/or rosin esters; terpenes derived, for example, from wood or citrus, etc.); perhaps waxes, plasticizers or other materials to modify viscosity (i.e., flowability) (examples of such materials include, but are not limited to, mineral oil, polybutene, paraffin oils, ester oils, and the like); and/or other additives including, but not limited to, antioxidants or other stabilizers. For example, a typical hot-melt adhesive formulation might contain from about 15 to about 35 weight percent cohesive strength polymer or polymers; from about 50 to about 65 weight percent resin or other tackifier or tackifiers; from more than zero to about 30 weight percent plasticizer or other viscosity modifier; and optionally less than about 1 weight percent stabilizer or other additive.

It should be understood that other adhesive formulations comprising different weight percentages of these components are possible. "Discontinuous bonding pattern" as used herein refers to a pattern of bonding areas, in particular bonding areas between layers, whereby at least in at least one region the layers are not bonded. A discontinuous bonding pattern may comprise a connected bonding area or multiple disconnected bonding areas. A discontinuous bonding pattern further may comprise a connected bonding area comprising a number of holes, where the layers are not bonded, preferably according to a regular pattern, or it may comprise discrete disconnected bonding areas, e.g. a point bonded pattern which comprises a plurality of separate bonding points surrounded by unbonded areas or a line-bonded pattern which comprises a plurality of separate bonding lines alternated by unbonded areas, preferably according to a regular pattern.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner) . As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length.

Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like. The term "elasticized" or "elastified" refers to a material, layer, or substrate that is naturally non-elastic, but which has been rendered elastic by, for example, suitably joining an elastic material, layer, or substrate thereto.

"Elongation" means the ratio of the extension of a material to the length of the material prior to the extension (expressed in percent). "Extension" means the change in length of a material due to stretching (expressed in units of length) .As used herein the term "extensible" means elongatable in at least one direction, but not necessarily recoverable.

The term "finished" or "final", when used with reference to a product, means that the product has been suitably manufactured for its intended purpose. As used herein, the term "garment" means any type of apparel which may be worn. This includes diapers, training pants, incontinence products, surgical gowns, industrial workwear and coveralls, undergarments, pants, shirts, jackets and the like.

The term "graphic" includes, but is not limited to, any type of design, image, mark, figure, codes, words, patterns, or the like. For a product such as a training pant, graphics will generally include objects associated with little boys and little girls, such as multi-color trucks, airplanes, balls, dolls, bows, or the like.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

"Integral" is used to refer to various portions of a single unitary element rather than separate structures bonded to or placed with or placed near one another. "Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

The term "laid-flat state" or "fully stretched state" or "extended state" is intended to refer to the article when it is flattened into a plane or is substantially flattened into a plane and is used in contrast to when the article otherwise positioned, such as when the article is folded or shaped in or for use by a wearer. The use of the term "layer" can refer, but is not limited, to any type of substrate, such as a woven web, nonwoven web, films, laminates, composites, elastomeric materials, or the like, or even formulations, such as adhesives, that form a substrate upon a change in conditions (e.g. solidification of a hotmelt adhesive when the temperature drops below a predetermined amount). A layer can be liquid and air permeable, permeable to air but impermeable to liquids, impermeable both to air and liquid, or the like. When used in the singular, it can have the dual meaning of a single element or a plurality of elements.

The term "nonwoven fabric or web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

Use of the term "substrate" includes, but is not limited to, woven or nonwoven webs, porous films, ink permeable films, paper, composite structures, or the like.

Superabsorbent materials (e.g. superabsorbent polymers) suitable for use in the present disclosure are known to those skilled in the art, and may be in any operative form, such as particulate form, fibers and mixtures thereof. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g. saline with 0.9 wt% NaCl). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like.

Other suitable hydrogel- forming polymers include hydrolyzed acrylonitrile grafted starch, acrylic acid grafted starch, and isobutylene maleic anhydride copolymers and mixtures thereof. The hydrogel- forming polymers may be lightly crosslinked to render the material substantially water insoluble. Crosslinking may, for example, be by irradiation or covalent, ionic, Van der Waals, or hydrogen bonding. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article of the present invention in an amount up to about 60% by weight.

Typically, the superabsorbent material, when present, will be included in an amount of about 10% to about 100% by weight, based on the total weight of the absorbent layer. "Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 µm, preferably between 300 to 600 µm, more preferably between 400 to 500 µm.

The term "topsheet" refers to a liquid permeable material sheet forming the inner cover of the absorbent article and which in use is placed in direct contact with the skin of the wearer. The top sheet is typically employed to help isolate the wearer's skin from liquids held in the absorbent structure. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g. urine or menstrual fluid. The inner coversheet may further be different in different parts of the absorbent article. The top sheet fabrics may be composed of a substantially hydrophobic material, and the hydrophobic material may optionally be treated with a surfactant or otherwise processed to impart a desired level of wettability and hydrophilicity.

As used herein, the term "transverse" or "lateral" refers to a line, axis, or direction which lies within the plane of the absorbent article and is generally perpendicular to the longitudinal direction.

"Ultrasonic welding" refers to a technology which joins two materials by melting them with heat generated from ultrasonic oscillation and then laminating them together, such that the molten materials flow and fill the gap between the two unaffected portions of the two materials, respectively. Upon cooling and shaping, the two materials are joined together.

"Form-driven locking" refers to locking via the shape of an element, such as a key lock wherein the shape of an element provides a resisting force to the opening thereof.

"Force-driven locking" refers to locking via a means selected from the group consisting of electromechanical, magnetic, adhesive and combinations thereof. "Substantial portion" as used herein with reference to a element/component (e.g. wetness sensing tracks), refers to at least 80%, preferably at least 90%, more preferably at least 95%, of said element/component, generally measured as a surface area taken in the laid flat state of the absorbent article.

"Wetness sensing tracks" as used herein refer to tracks that are suitable for wetness sensing but are not limited thereto, for example said tracks may further or alternatively sense other form of exudates that not necessarily result in wetness, such as stool detection. These can be configured to undergo changes in presence of certain substances, such as stool and/or cellulose fibers combined with superabsorbent polymers saturated with urine.

"Substantially perpendicular (or parallel)" refers to an element extending at an angle of not more than 35°, preferably less than 25°, more preferably less than 20°, even more preferably less than 15°, even more preferably less than 10°, most preferably less than 5°, from the referred perpendicular (or parallel) axis.

"Terminal" refers to the point at which a conductor from an electrical component, device or network comes to an end and provides a point of connection to external circuits. With this, terminals integrated into the clip-on data processing module as described herein are meant. These consist by preference of metal or a metal alloy, more preferably of a metal or metal alloy plated by a precious metal, even more preferably gold plated.

Embodiments according to the disclosure will now be described. It is understood that technical features described in one or more embodiments maybe combined with one or more other embodiments without departing from the intention of the disclosure and without generalization therefrom.

### THE SUBSTRATE

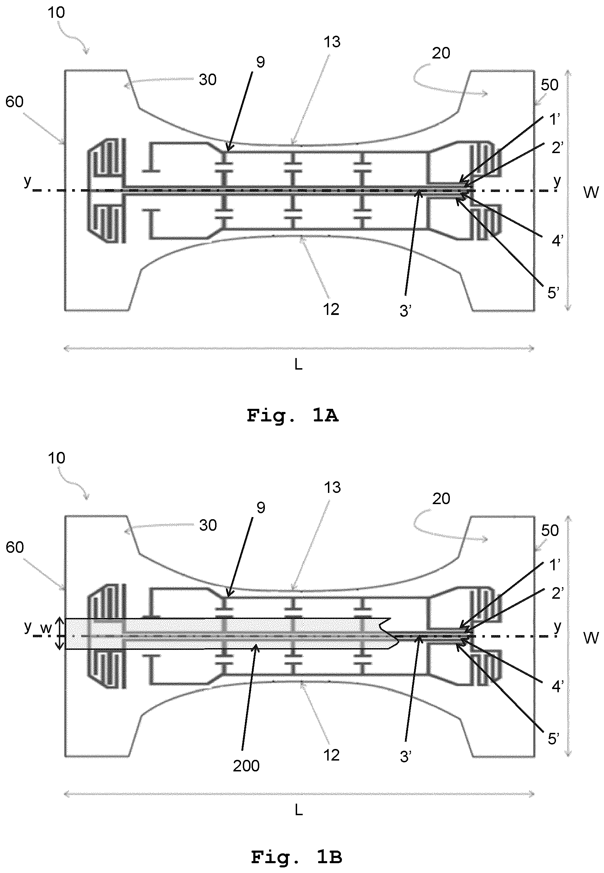

In an embodiment, exemplary shown in Figs. 1A to 1C, a substrate 10 suitable for incorporation into an absorbent article 100 for monitoring and detecting the presence of wetness events therein and/or risk of exudate leakage therefrom, is provided. Said substrate 10 comprises a conductive pattern 101 disposed on a first surface 20 capable of being arranged proximal to a body facing side of the absorbent article 100, wherein said conductive pattern 101 can be brought in electrical communication with a clip-on data processing module 103. Said conductive pattern 101 comprises: a plurality of connection tracks, exemplary connection tracks 1',2',3',4',5'; and a plurality of sensing tracks 9 connected to said connection tracks 1',2',3',4',5', and wherein the conductive pattern 101 is configured in a manner that addressing multiple combinations of said plurality of connection tracks, for example tracks 1',2',3',4',5', with corresponding terminals of the clip-on data processing module 103, exemplary terminals 1,2,3,4,5, result in multiple electrical circuit configurations for measuring resistance, impedance and/or capacitance therethrough.

In the context of Figs. 1A to 1C, the substrate 10 further comprises a second surface 30 opposite said first surface 20 and capable of being arranged proximal to a garment facing side of said absorbent article 100. In addition to this or as an alternative, the clip-on data processing module 103 can be in electrical communication with the conductive pattern 101 when connected at a position proximal to a first end 50 of the substrate 10, such to form at least one closed electrical circuit.

In a preferred embodiment, the connection tracks, for example tracks 1',2',3',4',5', are configured to allow an electrical current to flow from corresponding terminals of the clip-on data processing module 103, exemplary terminals 1,2,3,4,5, when connected thereto. Additionally or alternatively, said connection tracks may also be configured to allow an electrical current to flow to, or from, corresponding terminals of the clip-on data processing module 103 (sometimes also referred to hereinafter as clip-on module).

It may be beneficial that said connection tracks, exemplary tracks 1',2',3',4',5', extend substantially parallel to a length L of the substrate and substantially parallel to a longitudinal axis (y-y) crossing a first end 50 and a second end 60 of the substrate 10. Moreover, the sensing tracks 9 are configured to undergo changes in presence of certain substances, such as stool and/or cellulose fibers combined with superabsorbent polymers saturated with urine.

Advantageously, it has been found that a substrate comprising the above described conductive pattern 101 provides for accurate detection of exudates as well as being particularly suitable for fully scalable for in-line mass production at high speeds, without the need to change arrangement depending on product sizes. For example, an advantage of positioning the sensing tracks connected to the connection tracks is that if/when the substrate is cut to form for example leg openings (which may vary depending on product size), the circuitry is not damaged and wetness detection is still ensured across the areas of greater risk of leakage that are located proximal to the edges and/or sides of the absorbent article.

Further advantageously, the conductive pattern 101 mentioned above allows for multiple usage of terminals 33 of the clip-on data processing module 103, which in turn enables to detect wetness along different, specific zones of the absorbent article 100, as will be described in further detail later in the disclosure.

In an embodiment, at least one of the connection tracks, has the highest conductivity compared to the other connection tracks, and can be referred to as a feedback track. Said feedback track may extend along a centerline of the substrate 10 running along the longitudinal axis y-y. Preferably, the rest of the plurality of connection tracks are symmetrically disposed on either side of said central track.

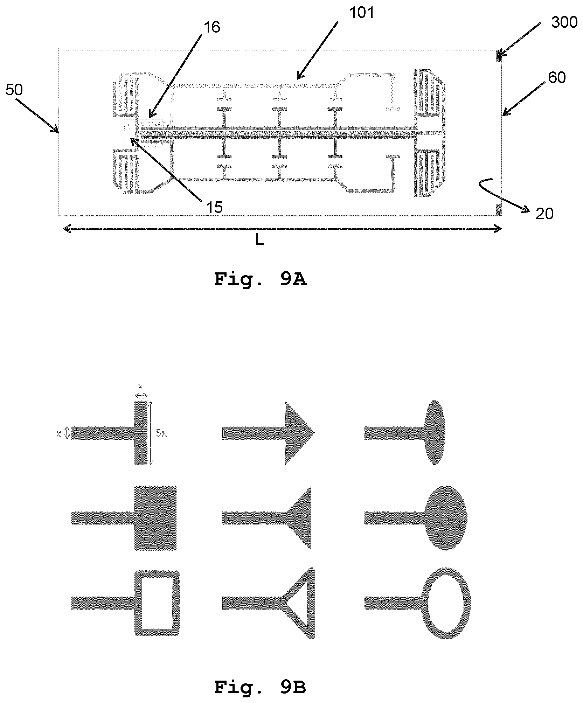

Preferably, the conductive pattern 101 comprises an electrically conductive material, and is preferably a printed conductive pattern, so that the distribution of said conductive pattern 101 (also referred to herein as sensor design) is registered and, hence, the sensor design is directly comprised on the substrate 10. This is advantageous, since further modifications as e.g. in order to characterize the absorbent product 100 (including for example information regarding the product size, absorbency of the core, colour of the product or the like), can be directly comprised within the printed conductive pattern. In addition to this, said sensor design may also comprise registration marks 300, as depicted in Fig. 9A, arranged to provide a trigger to a registering device to carry out process steps.

Furthermore, the conductive pattern 101 comprises, preferably consist of, a carbon-based ink and/or a conductive polymer-based ink, preferably the carbon-based ink comprising a conductive compound selected from the group consisting of graphene, graphite, nano-carbon-tubes and mixtures thereof, preferably the conductive polymer-based ink comprising a conductive compound selected from the group consisting of polyacetylene, polypyrrole, polyaniline and copolymers thereof, more preferably selected from the group consisting of poly(pyrrole)s (PPY), polyanilines (PANI), poly(thiophene)s (PT), poly(p-phenylene sulfide) (PPS), poly(p- phenylene) (PPP), Poly(acetylene)s (PAC), Poly(p-phenylene vinylene) (PPV), poly(3,4-ethylenedioxythiophene) (PEDOT), and mixtures thereof, most preferred conductive polymer-based ink comprising poly(3,4-ethylenedioxythiophene) polystyrene sulfonate (PEDOTPSS). This is advantageous since fast production is achieved whilst ensuring good compatibility with the end/final product unlike with the addition of foreign conductive elements like copper wires and the like, which are more prone to tearing the liquid impermeable substrate. Indeed, it is preferred in the disclosure herein to avoid metal containing inks (i.e. ink is free of metals) particularly due to the environmental impact to disposability.

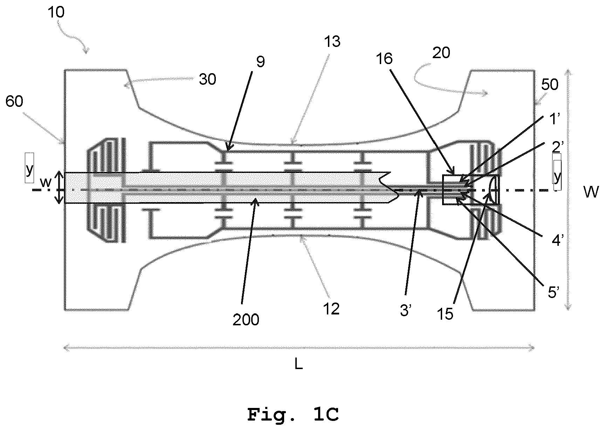

In a highly preferred embodiment, as depicted in Figs. 1B and 1C, the substrate 10 further comprises at least one insulating layer 200, preferably being liquid impermeable, adhered thereto and sized to cover at least a portion of the conductive pattern 101 (typically said "at least portion" being proximal to a position where the clip-on data processing module 103 is connected to said substrate). Said at least one insulating layer 200 is adapted to provide a seal and/or barrier to liquid and/or exudates from coming into contact with said portion of the conductive pattern 101, preferably wherein the sensing tracks 9 remain exposed to liquids and/or exudates and are not covered by said at least one insulating layer 200. Advantageously, the at least one insulating layer protects portions of the conductive pattern 101 to come into direct contact with exudates, limiting the risk of false positives, noise and even failure of detection.

Preferably, the at least one insulating layer 200 is a nonconductive substrate such as a film typically comprising polyethylene, alternatively the at least one insulating layer 200 is in the form of a non-conductive adhesive (preferably a hotmelt adhesive) and/or non-conductive ink.

Moreover, in an embodiment, the at least one insulating layer 200 has a length extending parallel to the length L of the substrate 10 that is equal or less than said length L of the substrate 10. Preferably, the length of said at least one insulating layer 200 is from 0.8L to L, preferably from 0.85L to 0.99L.

In a preferred embodiment, see Fig. 1B, the width w of the at least one insulating layer 200 is less than the width W of a backsheet (and/or substrate 10), preferably is less than 0.5W, more preferably from 0.05W to 0.35W, even more preferably from 0.08W to 0.30W, most preferably from 0.1W to 0.2W.

Moreover, in an embodiment, the at least one insulating layer 200 has a thickness (typically extending along an axis perpendicular to both the length and width w of said insulating layer) of 0.7 mm or less, preferably from 0.005 mm to 0.500 mm, more preferably from 0.010 mm to 0.200 mm, more preferably from 0.015 mm to 0.08 mm. Thickness measurements may be made according to ASTM D1777 and typically using a thickness tester such as C640, or CHY-C2A of Labthink International, 200 River's Edge Drive, Medford, MA, USA.

In a highly preferred embodiment, as depicted in Fig. 1C, the substrate 10 comprises one or more slits 15 for insertion of the clip-on module 103. In addition to this, a pocket 16 is formed between the first surface 20 and the at least one insulating layer 200 proximal to the first end 50, said pocket 16 being in fluid communication only with said slit(s) 15 and arranged to retain at least a portion of the clip-on data processing module 103 therein. This is beneficial, since an effective retaining pocket is attained in a cheap and scalable manufacturing process allowing for manufacturing of such substrates at high production speeds which still ensures a pocket is formed to retain the clip-on module in contact with the connection tracks whilst at the same time providing insulation against exudates as the substrate becomes saturated.

Preferably, the pocket 16 is sized such that it matches the dimensions of a pocket insertion member of the clip- module 103 (sometimes also referred to herein as the connection member) that typically comprises terminals 33 (as will be described herein below in more detail), such that said terminals 33 come into electrical communication with respective connection tracks by simply inserting the clip-on module 103 into the pocket 16 until a pocket dimension stops it from being inserted any further. Advantageously, this allows the caregiver to connect said clip-on module 103 without having to worry about particularly positioning the module to ensure good electrical connection.

For insertion of the module, an opening cut (or slit) can be created within the substrate. The opening cut can be a simple straight line or it can be a shape that requires to remove the cut out trim during the manufacturing process. The opening cut may be shaped in a way that it allows to identify easily the position for insertion of the clip-on module, which allows easy insertion and that can hold the module securely in place after insertion.

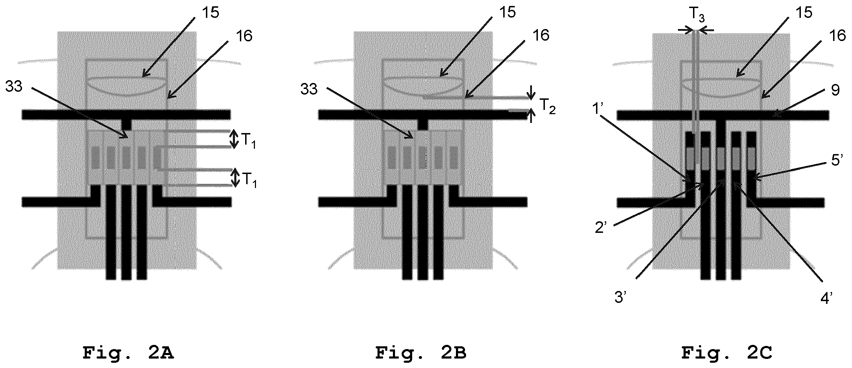

In a preferred embodiment, exemplified in Figs. 2A-2C, the sensor design, i.e. the conductive pattern 101, integrates tolerances T₁ for positioning of the clip-on data processing module 103 in a direction along an axis parallel to the longitudinal axis (y-y) and proximal towards the first end 50, which counteracts both the positioning of glue when the substrate 10 is integrated in the absorbent article 100, and manual attachment of the re-usable clip-on module 103 to the absorbent article 100 in the same direction. Additionally, the sensor design integrates a tolerance T₂ for positioning of the slit(s) 15 in a direction along an axis parallel to the longitudinal axis (y-y) with respect to the placement of the conductive pattern 101 onto the first surface 20. This arrangement counteracts both the placement of the conductive pattern 101 (for example through camera detection of eyemarks used when positioning the registered pattern against the substrate 10) and the positioning of the slits(s) 15 which would damage the printed sensor if made much closer to the conductive pattern, see Fig. 2B.

Furthermore, said conductive pattern also integrates a tolerance T₃ for positioning of the clip-on data processing module 103, and hence of its terminals 33, with respect to the position of the connection tracks, exemplary connection tracks 1',2',3',4',5' in Fig. 2C. This arrangement counteracts placement of the printed sensor against the substrate 10, application of glue in a direction perpendicular to the longitudinal axis (y-y), and manual attachment of the re-usable clip-on module 103 to the absorbent article 100 in said direction.

The conductive pattern 101 extends symmetrically in a direction substantially parallel to the length L of the substrate 10 and in a direction substantially perpendicular thereto, defining a plurality of independent electrical circuits, and wherein combinations of said electrical circuits are in electrical communication by selectively addressing at least two of the connection tracks with at least two corresponding terminals 33 of the clip-on data processing module 103. Preferably, the number of terminals 33 of the clip-on module 103 is at least the same as the number of connection tracks.

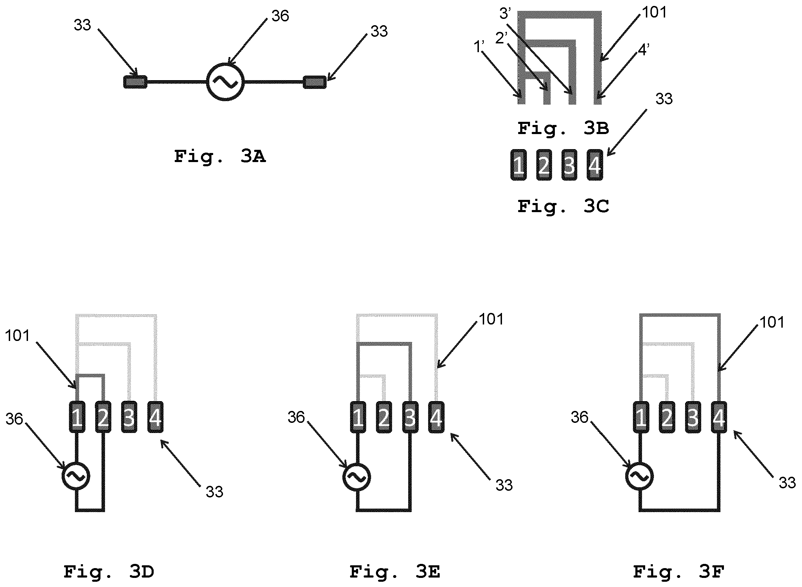

The basic concept in which the plurality of connection tracks can be selectively addressed by multiple terminals 33 of the clip-on module 103 is described in the context of Figs 3A to 3F. Preferably, at least two terminals 33 of the clip-on data processing module 103 can be connected to a voltage source 36, as illustrated in Fig. 3A. The voltage source 36 may be part of a conductive circuit that incorporates at least a portion of the conductive pattern 101. Alternatively, the voltage source 36 may be part of a circuit incorporated into the terminals 33 of the clip-on module 103. Fig. 3B depicts an example of a (printed) conductive pattern 101 comprising a plurality of connecting tracks, exemplary four tracks 1',2',3',4', which can be connected to corresponding available terminals, for example 1,2,3,4, of the clip-on module 103 when attached thereto, shown in Fig. 3C.

Figure 3D shows an exemplary circuit that is activated upon connecting the voltage source 36 to terminals 1 and 2 of the clip-on module 103, which in turn are connected to corresponding connection tracks 1' and 2'. From Figs. 3C to 3E it can be noted that even though several tracks of the conductive pattern 101 are available, different circuits are activated by selectively addressing (hereinafter both "addressing" and "connecting" can be used to refer to the same mechanism), via the voltage source 36, at least two terminals of the clip-on module 103 to at least two corresponding connection tracks. For the sake of clarity, hereinafter the voltage source 36 may not be explicitly mentioned (unless necessary) when referring to "terminals 33 connected to connection tracks".

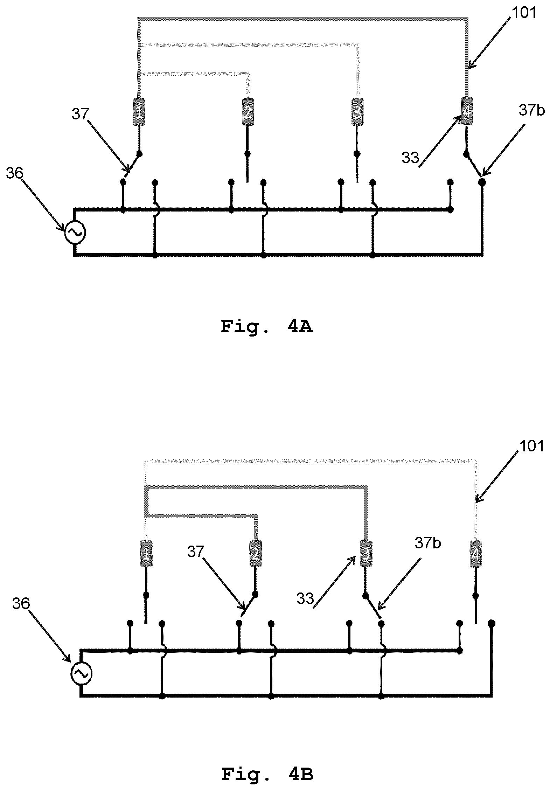

The selection of said at least two of the plurality of connection tracks and/or of the terminals 33 may be made electronically. By way of example, such an electronic selection can be achieved by controlling available terminals 33 of the clip-on data processing module 101 via one or more switches 37, as illustrated in Figs. 4A and 4B in which an exemplary electrical circuit for an arrangement with a conductive pattern 101 comprising four connection tracks 1',2',3',4' and corresponding four terminals 1,2,3,4 of the clip-on module 103 are connected to a voltage source 36 trough switches 37. One part of said exemplary circuit may belong to the conductive pattern 101 whereas another part that comprises the voltage source 36 and switches 37 may be part of, or alternatively may be connected to, the terminals 1,2,3,4, of the clip-on module 103. In Fig. 4A, a circuit is enabled controlling terminals 1 and 4 by closing switches 37, 37b. A different circuit is enabled by selectively connecting terminals 2,3 via exemplary switches 37, 37b, to corresponding connection tracks 2',3', see Fig. 4B.

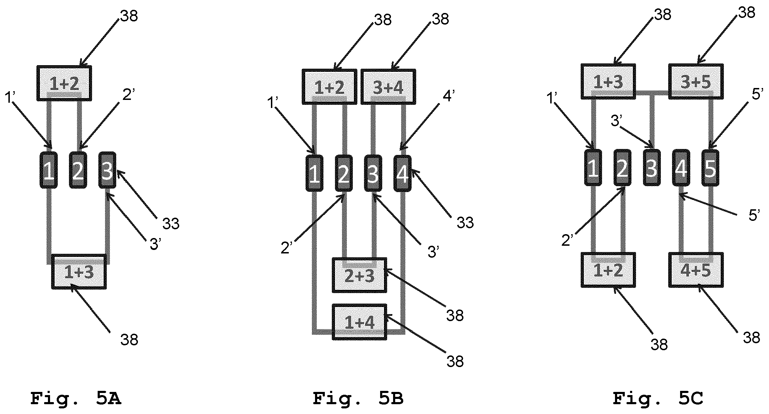

Also, combinations of circuits defined by the conductive pattern 101 may be enabled by selectively addressing multiple combinations of at least pairs of connection tracks by multiple combinations of corresponding pairs of terminals 33. By way of example, the circuit shown in the upper part of Fig. 5A is selectively activated or enabled by addressing connection tracks 1',2, by terminals 1,2, whilst another circuit in the lower part of Fig. 5A is selected by bringing terminals 1,3 in electrical contact with connection tracks 1',3'.

Moreover, by selectively addressing multiple combinations of said plurality of connection tracks with corresponding terminals of the clip-on data processing module 103, measurements of resistance, impedance and/or capacitance through the multiple electrical circuits defined by said conductive pattern 101 are also enabled.

Furthermore, as explained before, portions of the conductive pattern 101 may be designed in such a way that they are configured to undergo changes, preferably in its electrical properties, in presence of certain substances (such as stool and/or cellulose fibers with superabsorbent polymers saturated with urine, or the like) and as such are sensing tracks 9. Thus, variations in the measured resistance, impedance and/or capacitance through selected electrical circuits defined by said sensing tracks allow to detect whether at least a portion of said circuit is in contact with wetness due to exposure to liquids and/or exudates.

It is also mentioned that the present invention relates to the fact that conclusions on the presence of certain substances can be translated into the zone where the substance is actually present. Exemplary, Figs. 5A-5C illustrate that multiple combinations of multiple terminals 33 may also result in retrieving information about sensing zones, defined as zones on the substrate 10 at which the conductive pattern 101 is capable to undergo changes of resistance, impedance and/or capacitance. Especially, as can be observed in Fig. 5A, addressing terminals 1,2 by corresponding connection tracks 1',2' activates a circuit located in the upper zone 38 of said Fig. 5A. In the case that the portion of the conductive pattern 101 in said zone comprises sensing tracks 9, a sensing zone 38 and depicted as zone "1+2" is therefore defined. In addition to this, the circuit enabled by applying a voltage source to exemplary terminals 1,3 addressing corresponding connection tracks 1',3' may also result in another wetness zone 38, depicted as zone "1+3".

Hence, one may also note that multiple combinations of available terminals 33 addressing corresponding connection tracks of the conductive pattern 101 can be chosen in order to detect specific and/or desired zones of the substrate 10 where wetness events are expected to occur. Figs. 5B and 5C illustrate exemplary arrangements with multiple combinations of at least two terminals 33 selectively addressing at least two connection tracks of the (printed) conductive pattern 101, enabling various circuits and corresponding sensing zones.

The number of combinations of terminals that can be addressed to connection tracks of the substrate 101, can be calculated theoretically. For a given set of ***n*** terminals, the number of combinations of two terminals out of these ***n*** is given by given by *c_{theory}* obtained from the following equation: $\left(\begin{matrix}n \\ 2\end{matrix}\right) = \frac{n !}{2 ! \left(n-2\right) !}$

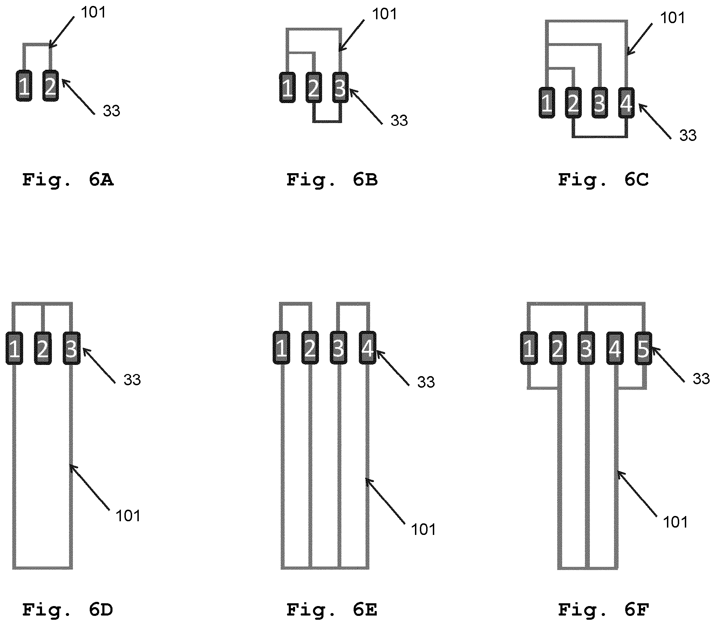

Nevertheless, design constraints related to working in a two-dimensional (2D) space of the substrate 10 are encountered, yielding in a limited number of combinations of the ***n*** terminals ***cᵣₑₐₗ*,** which are equal or lower than the expected number *c_{theory},* i.e. *cᵣₑₐₗ* ≤ *c_{theory}.* Whereas a combination of two terminals with two connection tracks is straight forward, as exemplary depicted in Fig. 6A, connecting two, three or more terminals becomes more complicated, since it requires to include additional conductive tracks into the design of the conductive pattern 101. Working within a 2D substrate 10 (onto which the conductive pattern is applied) requires that adding multiple tracks to the (printed) conductive pattern 101 for connecting more terminals should avoid creating crossing tracks. Such crossing tracks may create shortcuts within the conductive pattern that may leave some parts of the substrate 10 undetected by the terminals. Furthermore, the addition of conductive tracks in order to connect multiple pairs of terminals 33 may not result in a unique connection between said two terminals, but may rather define multiple circuits that could be activated by addressing said two terminals, as exemplary shown Figs. 6B and 6C. Hence, it might not be possible to define a unique sensing zone when addressing two connection tracks by corresponding terminals, via a voltage source 36.

The present invention advantageously circumvents this situation by designing the conductive pattern 101 in a manner that it defines multiple circuits which yield unique sensing zones when addressed by the clip-on module 103. Exemplary arrangements with three, four and five connection tracks addressed by corresponding three, four and five terminals are illustrated in Figs. 6D to 6E, respectively.

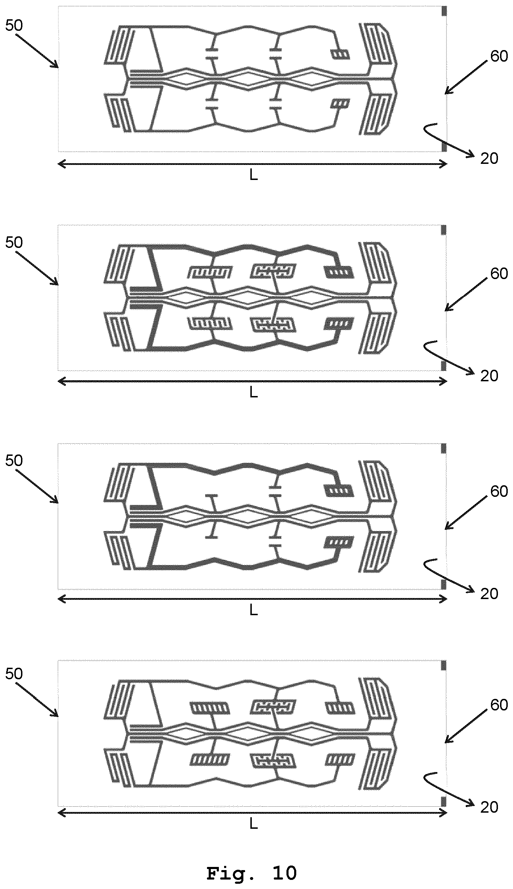

In a preferred embodiment, the conductive pattern 101 extends symmetrically in a direction substantially parallel to the length L of the substrate 10 and in a direction substantially perpendicular thereto defining a plurality of independent electrical circuits, and wherein combinations of said electrical circuits are in electrical communication by selectively addressing at least two of the connection tracks with at least two corresponding terminals of the clip-on data processing module 103, as explained before. Examples of such conductive patterns are shown in Figs. 9A and 10.

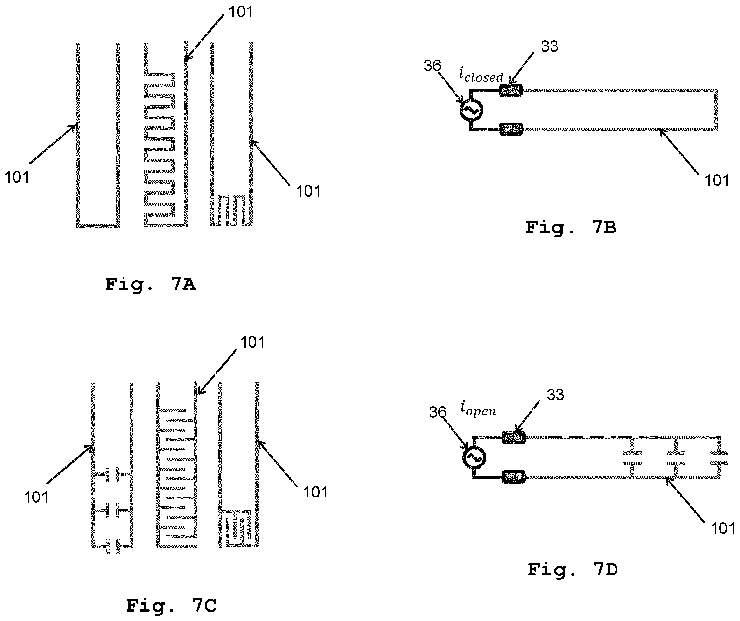

Preferably, said plurality of circuits defined by the conductive pattern 101 are circuits with a closed configuration, as exemplary shown in Fig. 7A. The plurality of circuits defined by the conductive pattern 101 comprise circuits with an open configuration, as exemplary depicted in Fig. 7C. Additionally or alternatively, said plurality of circuits defined by the conductive pattern 101 have a configuration that enables to apply glue lines when the substrate 10 is incorporated into an absorbent article 100, so that said glue lines do not harm the electrical properties of the circuits, as will be discussed later on.

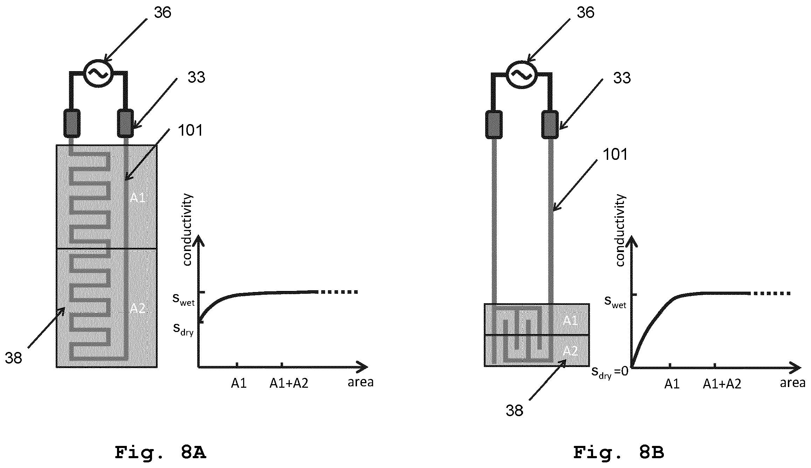

Closed circuits, as exemplary shown in Fig. 8A, are characterized by a finite conductivity ***S_{dry}*** measured upon applying a known voltage source 36 (additionally or alternatively a known current source ***i_{closed}***) by connecting at least two corresponding terminals 33 of the clip-on module. Open circuits are characterized by a conductivity equal to zero, ***S_{dry}* = 0,** since said circuit is not yet closed by connecting at least two terminals of the clip-on data processing module 103, see Fig. 8B. An advantage of open circuit configurations over closed circuit configurations is related to their electrical response to the presence of wetness, as illustrated also in Figs. 8A and 8B: once a wetness event occurs, a portion of the open circuit (Fig. 8A) and of the closed circuit (Fig. 8B) exposed to wetness define a first sensing zone 38 with area A1; a subsequent wetness event is considered, defining an enlarged sensing zone 38 with area A1+A2. This is reflected in the conductivity measured through said circuits as a function of the wet area. In the case that the circuits are not subject to wetness, a measured conductivity for the closed circuit starts from a finite value ***S_{dry}* ≠ 0,** whereas for an open circuit it starts at zero value, *S_{dry}* = 0. For both open and closed circuits, the conductivity increases as the wetness area increases and reaches a steady value ***S_{wet}.*** Such a steady conductivity ***S_{wet}*** may be in the same range for the closed circuit and for the open circuit configuration, as opposed to ***S_{dry}.***

The sensing capability for the circuits defined by the conductive pattern 101 is found by the difference on the conductivity values ***S_{wet} -S_{dry};*** hence, an open circuit configuration has a sensing capability higher than a sensing capability of a closed circuit configuration. This feature is particularly advantageous, since the measurements of resistance, impedance and/or conductivity of the various circuits defined by the conductive pattern 101 may be straight forward to interpret as a value of the sensing capability, or alternatively as the capability to detect wetness.

A disadvantage of an open circuit configuration over a closed circuit configuration is the lack of ability to verify whether the terminals 33 of the clip-on module 103 are properly connected to the (printed) connection tracks of the conductive pattern 101. Thus, it might be particularly advantageous that the plurality of circuits defined by the conductive pattern 101 comprises combinations of both closed circuit configurations and open circuit configurations, as exemplary shown in Figs. 9A and 10.

In a preferable embodiment, at least one of the plurality of circuits defined by the conductive pattern 101 is a circuit with a closed configuration, in order to ensure that a proper connectivity is achieved between the plurality of connection tracks of the conductive pattern 101 and corresponding terminals of the clip-on module 103 when attached thereto, see the example in Fig. 9A.

Moreover, the conductive pattern 101 may have a shape selected from straight lines and/or curved lines, exemplified in Fig. 10. Advantageously, said arrangements may circumvent some defects on the printing process placing the conductive pattern 101 onto the first surface 20 of the substrate 10.

In an embodiment, the sensing tracks 9 of the conductive pattern 101 may have a connected shape. These shapes may comprise, for example, solid or open rectangles and/or solid or open triangles and/or solid or open ellipses or a combination thereof, as shown in Fig. 9B. Preferably, sensing tracks 9 have a "T" shape with a height equal to the width of the sensing track and a width equal to 5 times the width of the sensing track.

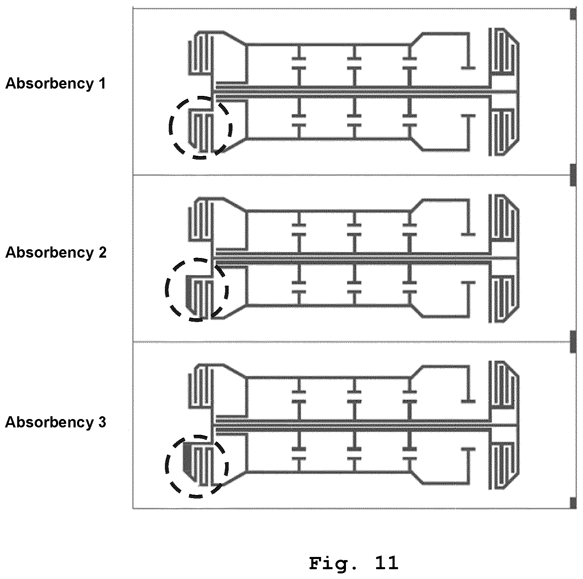

In an embodiment, a thickness and/or a length and/or a width and/or a specific conductivity of at least a portion of at least one of the plurality of circuits defined by the conductive pattern 101 comprises identifiers of the absorbent article 100 that are detected when connected to the clip-on data processing module 103. Preferably, said at least one circuit is a closed circuit configuration. Advantageously, identifiers of an absorbent article 100 (such as the level of absorbency and/or the size and/or the color and/or an inventory number or the like) can be predefined by assigning an initial capacitive and/or resistive measurement to a specific circuit of the conductive pattern 101, preferably being a closed circuit, upon incorporation to the absorbent article. Pre-set values may be stored in a memory within the clip-on data processing module 103. This is exemplified in Fig. 11, in which three conductive patterns 101 are shown, each having a closed circuit configuration with different thickness in its side which results in three different values for a measured resistance, impedance, and/or capacitance therethrough when measured by attaching the clip-on data processing module 103. Each of the measured resistance, impedance, and/or capacitance values can be related, for example, to a specific level of absorbency when the conductive pattern is incorporated to an absorbent article 100.

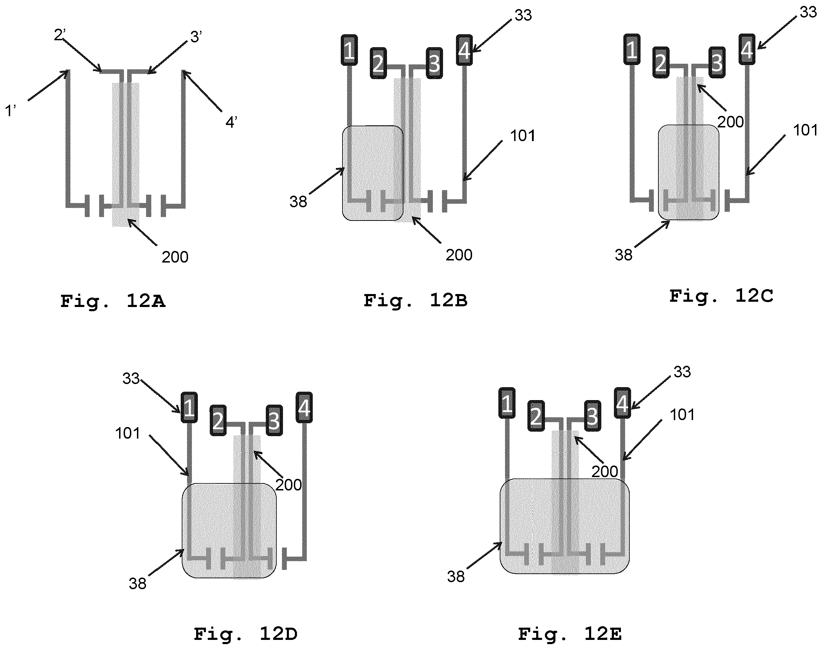

Further advantages of the present invention are shown in Figs. 12A-12E. In this context, it should be mentioned that enabling or activating open circuits with different configurations with respect to the position of the at least one insulating layer 200, results in specific response of the measured resistance, impedance and/or capacitance to the presence of wetness. Fig. 12A shows an exemplary embodiment comprising a combination of open circuit configurations for the conductive pattern 101 and one insulating layer 200.

If a wetness event occurs in the sensing zone 38 depicted in Fig. 12B, wetness may trigger a change in the resistance, impedance and/or conductivity (or generally electrical properties) of the circuit enabled by terminals 1 and 2. Additionally or alternatively, a wetness event present in a different sensing zone, exemplified in Fig. 12C, may trigger a change in the circuit connected by terminals 2 and 3. As a further example, see Fig. 12D, subsequent wetness events can activate an enlarged sensing zone 38, which triggers a change in the electrical properties of circuits enabled by terminals 1 and 2 addressing connection tracks 1',2', and 2 and 3 addressing connection tracks 2',3'. Subsequent wetness event may also trigger a change in the electrical properties of circuit enabled by terminals 3 and 4, addressing connection tracks 3',4'. It should also be mentioned that the sensing zones depicted in Figs. 12D and 12E may alternatively correspond to a single wetness event or alternatively to wetness accumulation instead of subsequent events.

Conversely, by selectively activating each of the circuits depicted in Figs. 12A-12E, it is possible to detect whether a corresponding sensing zone is wet or dry, i.e., whether the electrical properties of said circuits change at each sensing zone. This arrangement is particularly advantageous since it also allows to obtain a complete mapping of the wetness of the substrate 10, and of the absorbent article 100 in which it is incorporated.

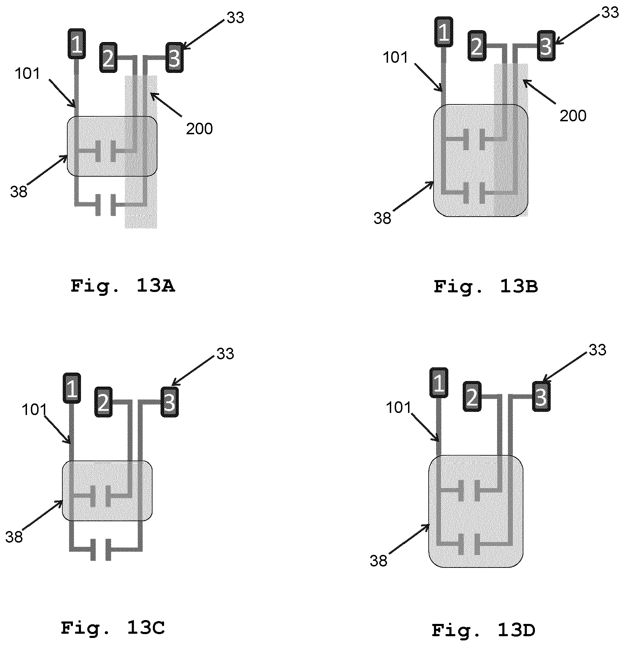

The electrical properties measured through multiple combinations of circuits defined by the conductive pattern 101 with respect to the presence of the at least one insulating layer 200 is illustrated in Figs. 13A to 13D. An example of two open circuit configurations enabled by thee connection tracks 1',2',3' and three terminals 1,2,3, defining a sensing zone 38, and comprising one insulating layer 200 is shown in Fig. 13A. Wetness occurring at said sensing zone 38 triggers a change only in the electrical properties of the circuit enabled by connecting terminals 1,2 to corresponding connection tracks 1',2'. It can be noted that a portion of the circuit enabled by terminals 1 and 3 is covered by the insulating layer 200, therefore said portion of the conductive pattern 101 is not exposed to wetness.

In contrast, for the exemplary arrangement without said insulating layer 200, shown in Fig. 13B, wetness at sensing zone 38 triggers a change in the electrical properties of both, the circuit enabled by terminals 1 and 2 as well as the circuit enabled by terminals 1 and 3, since all portions of the conductive pattern 101 defining the circuits are exposed to wetness (and therefore correspond to a sensing tracks 9). Hence, the use of at least one insulating layer 200 improves the accuracy on detecting zones at which wetness occur. In contrast, subsequent wetness may not be accurately detected if at least one insulating layer is not comprised in the substrate 10, as illustrated in the examples of Figs 13C and 13D, in which both shorten and enlarged sensing zones trigger changes in the two exemplary circuits.

### THE CLIP-ON DATA PROCESSING MODULE

Clip-on data processing module (also referred to as module or clip-on module) for use herein are preferably of the refastenable and replaceable kind. This means that the modules are adapted to be joined to an absorbent article and subsequently decoupled therefrom once the absorbent article is to be disposed. Thus, the modules are adapted for multiple use and may be connected to a plurality of absorbent articles in sequence (i.e. one after the other) and to the same or different patient/subject. The modules may be cleaned and battery charged in between a plurality of absorbent article changes. The net result is that an efficient, cost-effective and environment-compliant solution is provided.

As mentioned in the embodiments herein, the clip-on data processing module typically has a part that can be inserted into a pocket of the absorbent article, a second part that remains outside said pocket and a third part that allows bending said device between the first and the second part. The bending part can be a mechanical hinge or flexible element that allows the relative movement between first and second part. It is generally arranged in a way that allows all parts to be cleaned properly after use and avoids gaps that can undesirably facilitate the growth of bacteria and germs. The outer surface material of the modules preferably is selected to provide low friction for the first part that is meant to be inserted, ideally a rounded shape and preferably a soft material for the part that remains outside the pocket.

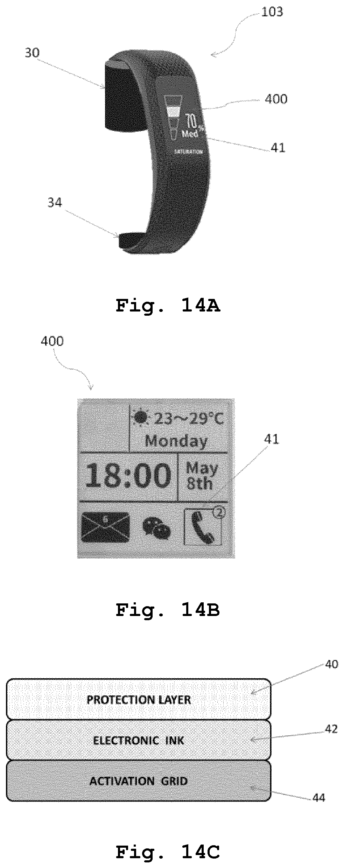

In the embodiments described above, a clip-on data processing module 103 suitable for removable attachment to a chassis of an absorbent article 100 for automatic detection of wetness therein and more preferably the risk of exudate leakage, is used. The module 103, illustrated in Fig. 14A, may comprise a housing 30 and a connection member 31 (the connection member used herein is preferably flexible but it is not necessary to be so, and rather also stiff members are suitable herein) coupled to said housing 30 and having a free end 32 being cantilevered from said housing 30, said end 32 comprising one or more electrically conducting connection ports (also referred to as terminals) 33, wherein the housing 30 comprises therein a data processing system, said data processing system comprising a power source, a processor, and a transmitter; and a motion sensor in electrical communication with at least said processor, the flexible connection member 31 being arranged to fasten to a surface of the absorbent article 100, wherein the absorbent article 100 comprises a plurality of sensor tracks 101, and to electrically connect at least two of said plurality of sensor tracks to said processing system via said one or more electrically conducting connection ports 33.

The motion sensor (herein also referred to as position sensor) comprises an accelerometer or gyroscope. The position data as measured by the module 103 may be combined with the resistance, impedance and/or capacitance measurements from said clip-on module 103 as described before to allow for a more accurate detection of wetness and saturation level at specific locations of the absorbent article. Advantageously, the present invention allows for an efficient electrical determination of wetness and sensing zones, so that the use of the motion sensor, implying to acquire more measurements and that also requires for interpretation of the measured results, is not needed.

The transmitter may be arranged to transmit/forward the data measured to a server or end-user, more preferably said data is transmitted to a server (for example a cloud-based server) wherein further processing of the data (although the processing may also be carried out at least in part also within the processing unit of the clip-on data processing module in the disclosure herein) via for example a mathematical logarithmic model is carried out for determination of patient related information, including risk of leakage within the absorbent article, and the further processed information is then uploaded on a graphical user interface to simply communicate to the caregiver a patient status, including providing a warning when the absorbent article is close to a point of leakage (e.g. above a predefined probability threshold such as above 80%, preferably above 85%, more preferably from 88% to 99%).

The power source comprised in the housing comprises a battery. The battery and the housing 30 can be disconnected from each other and from the module 103. Whenever the battery has to be replaced e.g. because it has a remaining charging capacity of only about 10% or less left, typically a waring is provided on a user interface to allow a caregiver to intervene and replace the battery by simply removing it from the housing 30 and replacing it with another (e.g. fully charged) one, while the measuring electronics (e.g. processor, motion sensor, sensor tracks, and the like) can continue performance under power via a micro-charger that is provided and connected thereto during the battery replacement. Time and money is saved whereas the entire module 103 does not have to be removed/replaced when only the low or flat battery has to be replaced. Alternatively, the battery may be an integral (non-disconnectable) portion of the unit within the housing.

The flexible connection member comprises an elastomer, and wherein the one or more electrically conducting connection ports are exposed from said elastomer when the clip-on data processing module 103 is not connected to the absorbent article 100. Preferably, the elastomer comprises one or more polymers having a monomer selected from the group consisting of carbon, silicone and mixtures thereof, preferably wherein the elastomer is selected to slide when contacted to PE. Advantages of this arrangement include improved comfort achieved upon connection as well as ease of cleaning after use.

The housing 30 and the free end of the flexible connection member comprise a locking member 34 to secure the clip-on data processing module 103 to the absorbent article 100, preferably said locking member 34 consisting of a form-driven locking member or a force-driven locking member and typically arranged to fasten the free end to a position proximal to a portion of the housing 30. Preferably, the locking member comprises a magnet arranged to provide a securing force between the free end and the housing 30 such that said free end adheres directly or indirectly, preferably indirectly via the backsheet of the absorbent article 100, to said housing.

Preferably, the flexible connection member has a width that is less than the width w of the at least one insulating layer 200 such that, when connected to the chassis of the absorbent article 100, said connection member slides between the at least one insulating layer 200 and the backsheet typically forming a pocket for receiving the same. An advantage of this arrangement is that a secure and protected connection may be achieved in a simple and cost-effective manner, resulting further in an optimum design for readily implementing in fast in-line process of manufacture.

As shown in Figs. 14A to 14C, the clip-on data processing module 103 may further comprise an electronic-ink based display 400 for displaying textual and/or graphical indicia 41 generally relating to the status of said module (such as battery level, wireless connection with the server and/or cloud and the like), patient information, and/or sensing status (such as saturation level of the absorbent article, % risk of leakage at any one point and the like), said display comprising: a layer of electronic ink 42 including a bi-stable non-volatile imaging material disposed between an activation layer (or activation grid) 44 and a transparent electrode layer located above the layer of electronic ink 42, for activating the layer of electronic ink 42 at particular locations to display textual and/or graphical indicia 41 on the surface of the display, wherein the layer of electronic ink 42 does not require electrical power to maintain the indicia 41 visible. Optionally, the electronic-ink based display 400 further comprises a protective layer 40 positioned such that the layer of electronic ink 42 is between said protective layer 40 and said activation layer 44.

In an embodiment, the display may comprise a rigid (e.g. glass-based thin-film-transistor (TFT)) or a flexible (e.g. plastic-based TFT) backplane. Exemplary commercially available displays having rigid backplane for use herein are for example ED013TC1, ED027TC2, ED029TC1, and/or ED035OC1 manufactured and sold by E Ink Corporation, a subsidiary of the YFY Group. Exemplary commercially available displays having flexible backplane for use herein are for example ET011TT2, ET013TT1, ET014TT1, ET029TC1, and/or ET014TT6 manufactured and sold by E Ink Corporation, a subsidiary of the YFY Group.

### THE ABSORBENT ARTICLE

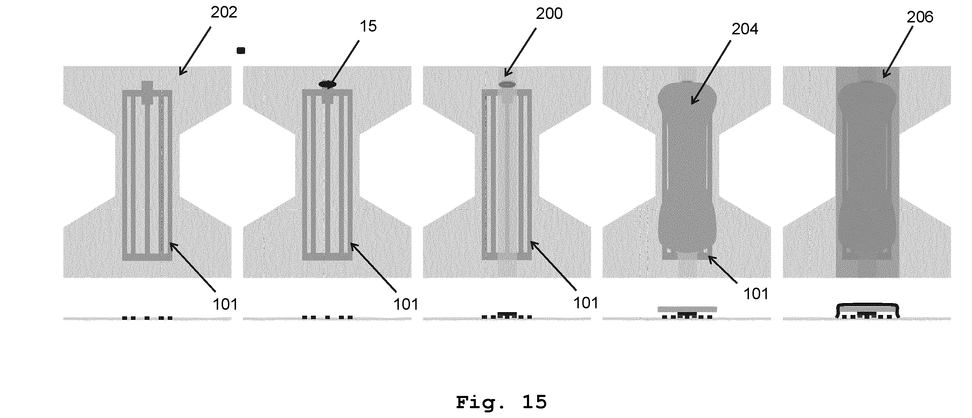

In an embodiment, exemplified in Fig. 15, an absorbent article 100 is preferably a disposable diaper, pad or pant, suitable for monitoring and detecting the presence of wetness therein and/or risk of exudate leakage therefrom, said absorbent article comprising: a liquid impermeable backsheet 202; a liquid permeable topsheet 206; an absorbent core 204 interposed between said backsheet 202 and topsheet 206, wherein said backsheet 202 comprises a substrate 10 described herein, typically the backsheet 202, absorbent core 204 and topsheet 206 forming a chassis of the absorbent article.

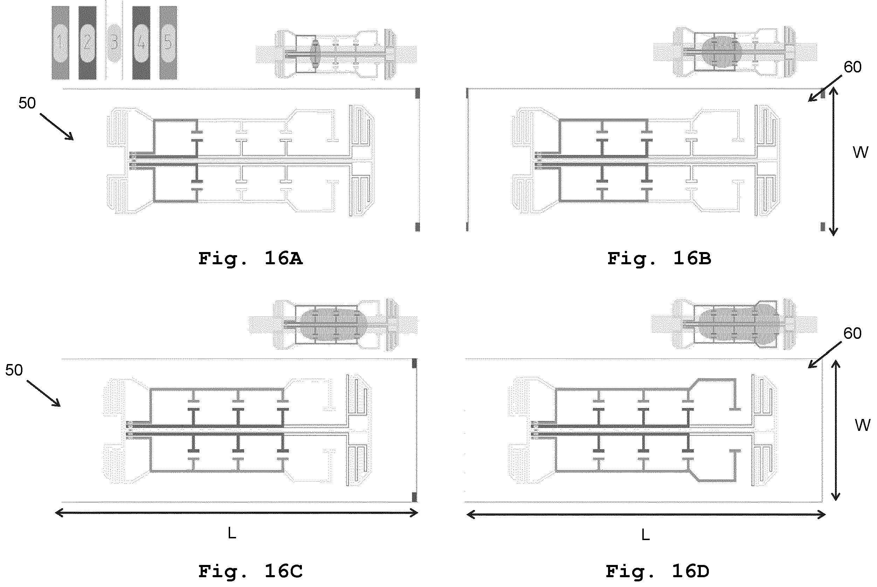

Preferably, the absorbent article further comprises a removable clip-on data processing module 103, as depicted in Figs. 16A to 16D, having a plurality of exposed electrically conductive terminals 33 capable of selectively address a plurality of connection tracks of a conductive pattern 101, preferably wherein the number of said terminals 33 is at least the same as the number of said connection tracks. By way of example, the terminals may be sized in sufficient length to ensure appropriate connection with the connection tracks.

Preferably, the absorbent article further comprises one or more slits 15 and/or pocket 16 on the backsheet 202 enabling an electrically conductive portion of said clip-on data processing module 103 to directly come in electrical communication with said connecting tracks.

In a preferred embodiment, at least a portion of the clip-on data processing module 103 is retained within a pocket 16, said pocket positioned over at least a portion of the absorbent core with the core being interposed between said pocket 16 and the skin of a subject when the absorbent article is worn such that when the clip-on data processing module 103 is inserted into the pocket 16 the absorbent core provides a cushioning layer between said clip-on data processing module 103 and said skin of the subject.

Preferably, the substrate 10 is positioned such that the first surface 20 faces the absorbent core 204, and preferably wherein the at least one insulating layer 200 is joined to said substrate 10 such that a liquid impermeable seal is formed providing a barrier to liquids and/or exudates expelled by a subject, when wearing the absorbent article, from coming into direct contact with at least a portion of the conductive pattern 101. It is highly preferred that at least a substantial portion of the sensing tracks 9 remains exposed to said liquids and/or exudates and not covered by said at least one insulating layer 200.

The absorbent core 204 may preferably comprise a nonwoven core wrap typically arranged such that there is no folding (i.e. no nonwoven overlap) of said core wrap coming in direct contact with the backsheet 202 comprising the conductive pattern 101. The nonwoven core wrap has typically hydrophilic properties, so that is allows the liquid to get in contact to the sensing tracks 9. Preferably, the backsheet 202 may comprise a polymeric film and/or a polymeric film laminated to a nonwoven (typically referred to a textile backsheet). The topsheet 206 preferably comprises nonwoven.

The absorbent article may further comprise additional components that are common in the art and selected from the group consisting of: a liquid distribution layer (ADL) positioned between the topsheet 206 and the absorbent core 202; elastic or non-elastic back ears joined to the chassis at a position proximal to the second end 60 of the substrate 10 forming the backsheet 202; super absorbent polymer particles and/or fibers typically comprised within the absorbent core; cellulose fibers typically comprised within the absorbent core 204; fastening tapes joined to the back ears for adhesive and/or mechanical coupling with a garment facing surface of the backsheet 202 at a position proximal to the first end 50; elastic waistband positioned proximal to the first and/or second ends 50,60; and combinations thereof.

When the absorbent article is a pant, the absorbent article may comprise a front belt positioned proximal to the first end 50 and a rear belt positioned proximal to the second end 60, said belts being separated from each other by the chassis of the absorbent article and being directly joined to each other via two opposite side seams, said belts typically being elastic. When the absorbent article is a pad it may be designed as a two-piece system, where the second piece is an elastic pant (reusable or disposable) to hold the first piece - said absorbent article - securely in place.

In a preferred embodiment, the first end 50 of the substrate (and/or backsheet 202) corresponds to the front (or belly portion) of the absorbent article and the second end 60 corresponds to the back of the absorbent article.

In a preferred embodiment, selectively addressing multiple combinations of the plurality of connection tracks with corresponding terminals of the clip-on data processing module 103 result in multiple electrical circuit configurations for measuring resistance, impedance and/or capacitance therethrough, as explained before for embodiments of the substrate 10.

Furthermore, measuring resistance, impedance and/or capacitance of multiple circuit configurations defined by the conductive pattern 101 selectively addressed with corresponding terminals of the clip-on data processing module 103, enables monitoring wetness at different zones of the core of the absorbent article 100; additionally or alternatively, enables monitoring a distribution of wetness in dependence of the actual and/or previous body position of a wearer of said absorbent article 100.

Moreover, identifiers of the absorbent article 100 can be detected when connected to the clip-on data processing module 103, based on a thickness and/or a length and/or a width and/or a specific conductivity of at least a portion of at least one of the connection tracks and/or the sensing tracks of the conductive pattern. Such identifiers may comprise, for example, level of absorbency and/or size and/or color and/or an inventory number of the absorbent article 100, or the like.

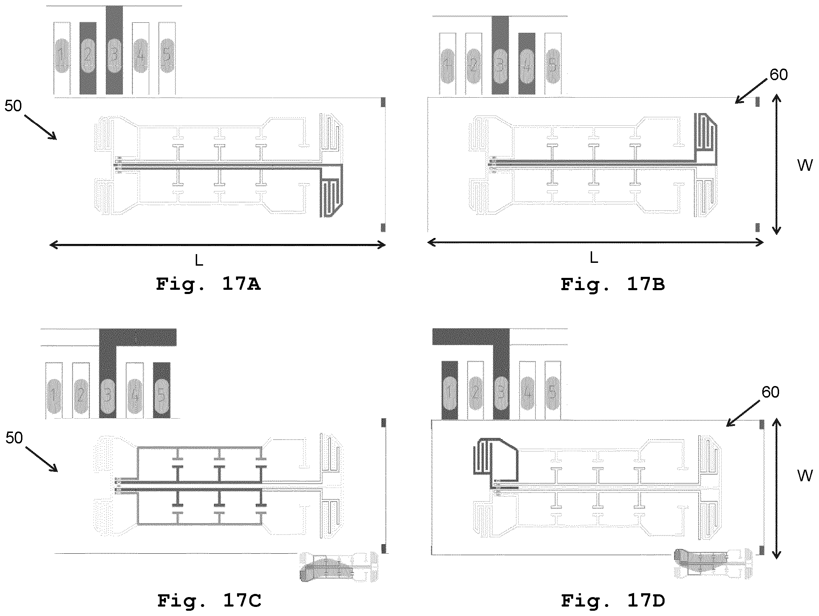

In addition to the explanations above, the preferred embodiments may be better understood with respect to the exemplary cases shown in Figs. 16A to 16D and Figs. 17A to 17D, in which the conductive pattern 101 of the substrate 10 (or backsheet 202) comprises five connection tracks, exemplary tracks 1',2',3',4',5', and the clip-on module 103 comprises five corresponding terminals, 1,2,3,4,5. Said conductive pattern 101 having, for example, a configuration comprising a combination of open circuit configurations and closed circuit configurations, as can be seen in Figs. 16A to 16D and 17A to 17D.

By selectively addressing connection tracks 2',4' with terminals 2,4, two open circuits located in the central part of the conductive pattern, and hence of the absorbent article 100, are enabled, whilst by selectively addressing connection tracks 1' and 5' with terminals 2 and 5, two open circuits located towards edges 12,13 of said substrate 10 are enabled, see Fig. 16A. In this manner, corresponding sensing zones 38 are defined, which in turn are located between the first end 50 and the second end 60 of the substrate 10, proximal to said firs end, and between edges 12 and 13 of the absorbent article 100 when worn by a subject, as depicted in the inset of Fig. 16A. Subsequent selective address of connection tracks 2',4' with corresponding terminals 2,4, and connection tracks 1',5' with corresponding terminals 1,5, enable further circuits and corresponding sensing zones 38 as depicted in Figs. 16B to 16C. Especially, it can be observed that with the configuration of the conductive pattern 101 used in this example, the sensing zones generated by selectively activating the circuits shown in Fig. 16D, extend over a zone perpendicular to the length L of the conductive pattern and hence of the absorbent article 100, so that the sensing zones are closely adapted to a body shape when worn by a subject.

Furthermore, terminals 2,3 also selectively address connection tracks 2',3' in order to activate the circuit shown in Fig. 17A, which in turn defines a sensing zone 38 that extends in a direction along the length L of the substrate and perpendicular thereto at a position proximal to the second end 60. Therefore, such a sensing zone enables to detect wetness occurring towards the back right end of the absorbent article when worn by a subject. Accordingly, selective addressing connection tracks 3',4' with terminals 3,4 enables activation of a circuit that defines a sensing zone located along an axis longitudinal to the axis (y-y) and at the back left end of the absorbent article as depicted in Fig. 17B. Moreover, selective activation of circuits enabling sensing zones in front left part and in front right part of the absorbent article are respectively shown in Figs. 17C and 17D. It is noted that the circuit exemplified in Fig. 17C is a closed circuit configuration which may also be used for product identification based on a thickness and/or a length and/or a width and/or a specific conductivity of at least one of the tracks defining such a closed circuit, as explained in previous embodiments.

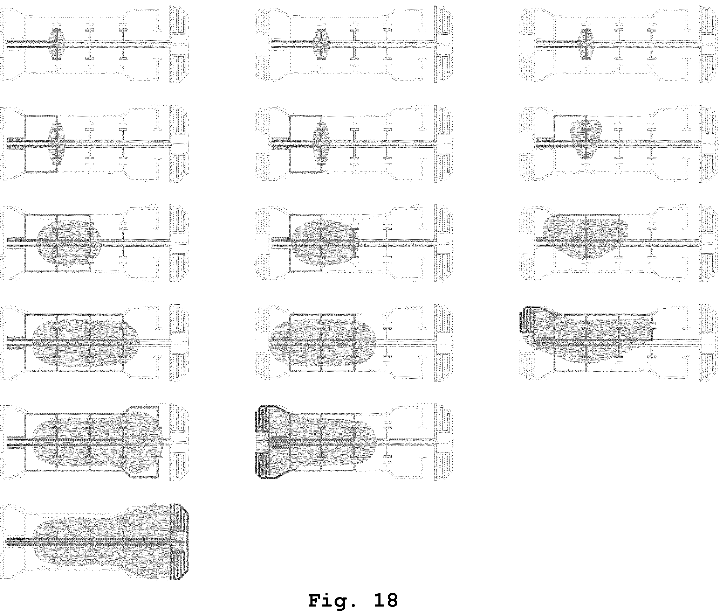

In this manner, by selectively activating combinations of multiple circuits of the conductive pattern 101, it is possible to generate a mapping of the wetness along the absorbent article 100, exemplary shown in Fig. 18. Preferably, the clip-on data processing module 103 is configured to process the measured resistance, impedance and/or capacitance by multiple combinations of terminals in order to generate said mapping of wetness. Additionally, the clip-on data processing module can also be adapted to generate and provide a warning to the care giver when the measured electrical properties in at least one of the sensing zones reaches a specific value, indicating that the absorbent article must be replaced. Such a value can be preset and may be stored in a memory within the clip-on data processing module 103.

Conversely, the example explained above in the context of Figs. 16A-16D and Figs. 17A-17D, also allows for detecting and monitoring wetness while occurring in the absorbent article 100: if wetness (due to liquids and/or exudates) is in contact to the conductive pattern 101 in the sensing zone shown in Fig. 16A, it triggers a change in the electrical properties of a circuit defining that zone, which is detected by terminals 2 and 4 of the clip-on data processing module 103 when attached thereto. Subsequent wetness areas trigger specific circuits defined by the conductive pattern 101, exemplary the circuits and corresponding zones depicted in Figs. 16B to 16D and Figs. 17A to 17D, which in turn can be detected by dedicated pairs of terminals 33.

Advantageously, resistance, impedance and/or capacitance measured through the multiple circuits defined by the conductive pattern 101, and the wetness detected through corresponding sensing zones can be also directly related to body position of the wearer, as e.g. a sensing zone detecting wetness in a location placed at the central part of the absorbent article 100 and close to its right edge, can be directly interpreted as wetness due to a wearer laying on his right side. Advantageously, efficiency and accuracy of the present invention is enhanced, since it is not necessary to combine the measured electrical data (resistance, impedance and/or conductivity) with measurements performed by the motion sensor of the clip-on module 103, resulting in less amount of data to be interpreted. Furthermore, the latter measurements can also be taken into consideration in order to provide a more precise mapping of wetness or mapping of body position if required.

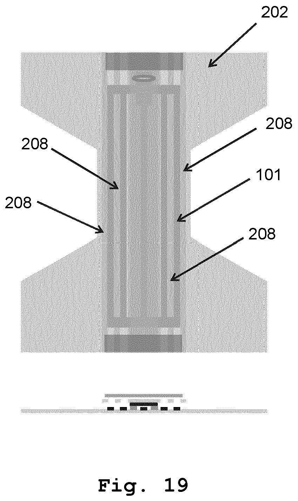

In an alternative embodiment, adapted glue pattern to laminate the body facing side of the backsheet 202 to the constituent layers can be applied. Said glue pattern comprises separated longitudinal lines 208 which leave parts of the conductive pattern 101 exposed, exemplified in Fig. 19. An advantage of this arrangement is that it allows using the conductive pattern 101 as disclosed herein without harming the configuration of the circuits defined therein.

### THE PROCESS

The disclosure herein further contemplates a method for making an absorbent article comprising the steps of: providing a liquid impermeable backsheet 202 and applying a conductive pattern 101 as described herein to a first surface thereof; providing and adhering at least one insulating layer 200 to said backsheet 202, said insulating layer being sized and positioned to cover at least a portion of the connection tracks of the conductive pattern 101, to provide a laminated substrate; providing an absorbent core 202 comprising absorbent material; providing a liquid permeable topsheet 206; and sandwiching the absorbent core 204 between said laminated substrate and said topsheet 206.

The method further comprises the step of providing a slit 15, additionally or alternatively a pocket 16, on the backsheet 202 enabling an electrically conductive portion of a clip-on data processing module 103 to selectively come in electrical communication with the connection tracks of the conductive pattern 101.

Preferably, the conductive pattern 101 is in the form of an electrically conductive ink and the application step comprises the printing thereof onto the backsheet. Moreover, the process generally comprises the step of detecting registered marks 300 to trigger accurate cutting of the substrate 10 (or the assembled/laminated continuous absorbent article 100) into a plurality discrete absorbent articles.

In a preferred embodiment the at least one insulating layer 200 is formed to have a width w, taken along an axis perpendicular to the longitudinal axis (y-y), being less than a width W of the backsheet 202 or substrate 10. Preferably, the width w is less than 0.5W, more preferably from 0.05W to 0.35W, even more preferably from 0.08W to 0.30W, most preferably from 0.1W to 0.2W.

In an embodiment, the layers referred to herein above (i.e. at least the backsheet 202 and the at least one insulating layer 200) are in the form of continuous webs and/or films that are first printed with conductive ink in the conductive pattern 101 described above prior to being laminated together and subsequently cut into individual absorbent articles.

In an embodiment, the at least one insulating layer 200 is adhered to the backsheet 202 by an adhesive and/or mechanical bonding, wherein the mechanical bonding is preferably selected from ultrasonic bonding, thermal bonding, and combinations thereof. In a preferred embodiment, the adhesive and/or mechanical bonding is applied across a length and width of the at least one insulating layer 200 in an effective amount such that bonding is achieved with the backsheet 202 and a liquid impermeable seal is formed providing a barrier to exudates expelled by a subject (when wearing the absorbent article 100) from coming into direct contact with at least a portion of the conductive pattern 101, preferably wherein at a location proximal to the first end 50 of the backsheet 202, where the clip-on module 103 is to be connected the adhesive and is present only outboard of the connection tracks and not therebetween such to form a pocket 16 for receiving an electrically conducting portion of said module 103.

In a preferred embodiment, monitoring and detecting the presence of wetness at different zones of the core of the absorbent article 100 is enabled by measuring resistance, impedance and/or capacitance of multiple circuit configurations defined by the conductive pattern 101 and selectively addressed by corresponding terminals 33 of the clip on module 103.

It is supposed that the present invention is not restricted to any form of realization described previously and that some modifications can be added to the presented example of fabrication without reappraisal of the appended claims.

## Claims

1. A substrate (10) suitable for incorporation into an absorbent article (100) for monitoring and detecting the presence of wetness therein and/or risk of exudate leakage therefrom, said substrate (10) comprising a conductive pattern (101) disposed on a first surface (20) capable of being arranged proximal to a body facing side of the absorbent article (100), wherein said conductive pattern (101) can be brought in electrical communication with a clip-on data processing module (103), said conductive pattern (101) comprising a plurality of connection tracks (1',2',3',4',5'); and a plurality of sensing tracks (9) connected to said connection tracks (1',2',3',4',5'),
**characterized in that**
the conductive pattern (101) is configured in a manner that addressing multiple combinations of said plurality of connection tracks (1',2',3',4',5') with corresponding terminals (1,2,3,4,5) of the clip-on data processing module (103) result in multiple electrical circuit configurations for measuring resistance, impedance and/or capacitance therethrough, and wherein the plurality of circuits defined by the conductive pattern (101) comprise circuits with an open configuration, and wherein the conductive pattern (101) extends symmetrically in a direction substantially parallel to the length (L) of the substrate (10) and a direction substantially perpendicular thereto, defining a plurality of independent electrical circuits, and wherein combinations of said electrical circuits are in electrical communication by selectively addressing at least two of the connection tracks (1', 2', 3', 4', 5') with at least two corresponding terminals (1, 2, 3, 4, 5) of the clip-on data processing module (103), and wherein resistance, impedance and/or capacitance measured through said plurality of circuits determines the wetness through corresponding sensing zones that are further directly related to body position of the wearer.-

2. The substrate (10) according to claim 1,
wherein the substrate (10) comprises a second surface (30) opposite said first surface (20) and capable of being arranged proximal to a garment facing side of said absorbent article (100); and/or
wherein the clip-on data processing module (103) is in electrical communication with the conductive pattern (101) when connected at a position proximal to a first end (50) of the substrate (10) such to form at least one closed electrical circuit.

3. The substrate (10) according to claim 1 or claim 2,
wherein the conductive pattern (101) comprises an electrically conductive material, and is preferably a printed conductive pattern, and
wherein the printed conductive pattern (101) comprises, preferably consists of, a carbon-based ink and/or a conductive polymer-based ink, preferably the carbon-based ink comprising a conductive compound selected from the group consisting of graphene, graphite, nano-carbon-tubes and mixtures thereof, preferably the conductive polymer-based ink comprising a conductive compound selected from the group consisting of polyacetylene, polypyrrole, polyaniline and copolymers thereof, more preferably selected from the group consisting of poly(pyrrole)s (PPY), polyanilines (PANI), poly(thiophene)s (PT), poly(p-phenylene sulfide) (PPS), poly(p-phenylene) (PPP), Poly(acetylene)s (PAC), Poly(p-phenylene vinylene) (PPV), poly(3,4-ethylenedioxythiophene) (PEDOT), and mixtures thereof, most preferred conductive polymer-based ink comprising poly(3,4-ethylenedioxythiophene) polystyrene sulfonate (PEDOT:PSS).

4. The substrate (10) according to any of the preceding claims,
wherein said connection tracks (1',2',3',4',5') are configured to allow an electrical current to flow from corresponding terminals (1,2,3,4,5) of the clip-on data processing module (103) when connected to it and/or
wherein said connection tracks (1',2',3',4',5') extend substantially parallel to a length L of the substrate and substantially parallel to a longitudinal axis (y-y) crossing a first end (50) and a second end (60) of the substrate (10); and
wherein said sensing tracks (9) are configured to undergo changes in presence of certain substances.

5. The substrate (10) according to any of the preceding claims,
further comprising at least one insulating layer (200) preferably being liquid impermeable, adhered thereto and sized to cover at least a portion of the conductive pattern (101), said at least one insulating layer (200) adapted to provide a seal and/or barrier to liquid and/or exudates from coming into contact with said portion of the conductive pattern (101), preferably wherein the sensing tracks (9) remain exposed to liquids and/or exudates and are not covered by said at least one insulating layer (200).

6. The substrate (10) according to any of the preceding claims
wherein said substrate (10) comprises one or more slits (15), and
wherein a pocket (16) is formed between the first surface (20) and the at least one insulating layer (200) proximal to the first end (50), said pocket (16) being in fluid communication only with said slit(s) (15) and arranged to retain at least a portion of the clip-on data processing module (103) therein.

7. The substrate (10) according to any of the preceding claims,
wherein the plurality of circuits defined by the conductive pattern (101) comprise circuits with a closed configuration and/or
wherein at least one of the plurality of circuits defined by the conductive pattern (101) is a circuit with a closed configuration, and/or
wherein the plurality of circuits defined by the conductive pattern (101) have a configuration that allows to apply glue lines when incorporated into an absorbent article (100).

8. The substrate (10) according to any of the preceding claims,
wherein a thickness and/or a length and/or a width and/or a specific conductivity of at least a portion of at least one of the plurality of circuits defined by the conductive pattern (101) comprises identifiers of the absorbent article (100) that are detected when connected to the clip-on data processing module (103).

9. An absorbent article (100), preferably a disposable diaper, pad or pant, suitable for monitoring and detecting the presence of wetness therein and/or risk of exudate leakage therefrom, said absorbent article comprising:
a liquid impermeable backsheet (202);
a liquid permeable topsheet (206);
an absorbent core (204) interposed between said backsheet (202) and topsheet (206), wherein said backsheet (201) comprises a substrate (10) according to any of the preceding claims.

10. The absorbent article (100) according to claim 10,
wherein said absorbent article further comprises a removable clip-on data processing module (103) having a plurality of exposed electrically conductive terminals (33) capable of selectively addressing a plurality of connection tracks (1',2',3',4',5') of a conductive pattern (101), preferably wherein the number of said terminals (33) is at least the same as the number of said connection tracks (1',2',3',4',5').

11. The absorbent article (100) according to claim 11,
wherein selectively addressing multiple combinations of the plurality of connection tracks (1',2',3',4',5') with corresponding terminals (1,2,3,4,5) of the clip-on data processing module (103) result in multiple electrical circuit configurations for measuring resistance, impedance and/or capacitance therethrough; and/or
wherein at least a portion of the clip-on data processing module (103) is retained within a pocket (16), said pocket positioned over at least a portion of the absorbent core with the core being interposed between said pocket (16) and the skin of a subject when the absorbent article is worn such that when the clip-on data processing module (103) is inserted into the pocket (16) the absorbent core provides a cushioning layer between said clip-on data processing module (103) and said skin of the subject.

12. The absorbent article (100) according to any of claims 9 to 11,
wherein measuring resistance, impedance and/or capacitance of multiple circuit configurations defined by the conductive pattern (101) selectively addressed with corresponding terminals (1,2,3,4,5) of the clip-on data processing module (103) enables monitoring wetness at different zones of the core of the absorbent article (100) and/or monitoring a distribution of wetness in dependence of the actual and/or previous body position of a wearer of said absorbent article (100).

13. A method for making an absorbent article according to any of claims 9 to 12 comprising the steps of:
providing a liquid impermeable backsheet and applying a conductive pattern (101), according to any of claims 1 to 8, to a first surface thereof;
providing and adhering at least one insulating layer (200) to said backsheet, said insulating layer being sized and positioned to cover at least a portion of the connection tracks (1',2',3',4',5') of the conductive pattern (101), to provide a laminated substrate;
providing an absorbent core comprising absorbent material;
providing a liquid permeable topsheet; and
sandwiching the absorbent core between said laminated substrate and said topsheet.

14. The method according to claim 13,
further comprising the step of providing a slit (15) and/or pocket (16) on the backsheet enabling an electrically conductive portion of a clip-on data processing module (103) to selectively come in electrical communication with the connection tracks (1',2',3',4',5') of the conductive pattern (101).

15. The method according to claims 13 or 14,
wherein the conductive pattern (101) is in the form of an electrically conductive ink and the application step comprises the printing thereof onto the backsheet; and/or
wherein the at least one insulating layer (200) is formed to have a width (w), taken along an axis perpendicular to the longitudinal axis (y-y), being less than a width (W) of the backsheet; and/or
wherein monitoring and detecting the presence of wetness at different zones of the core of the absorbent article (100) is enabled by measuring resistance, impedance and/or capacitance of multiple circuit configurations defined by the conductive pattern (101) and selectively addressed by corresponding terminals (33) of the clip-on data processing module (103).

## Patentansprüche

1. Ein Substrat (10), das zur Einarbeitung in einen absorbierenden Artikel (100) zur Überwachung und Erkennung des Vorhandenseins von Nässe darin und/oder des Risikos des Austritts von Exsudat daraus geeignet ist, wobei das besagte Substrat (10) ein leitfähiges Muster (101) aufweist, das auf einer ersten Oberfläche (20) angeordnet ist, die proximal zu einer dem Körper zugewandten Seite des absorbierenden Artikels (100) angeordnet werden kann, wobei das besagte leitfähige Muster (101) mit einem aufsteckbaren Datenverarbeitungsmodul (103) in elektrische Verbindung gebracht werden kann, wobei das besagte leitfähige Muster (101) eine Vielzahl von Verbindungsbahnen (1',2',3',4',5') und eine Vielzahl von Messbahnen (9) umfasst, die mit den besagten Verbindungsbahnen (1',2',3',4',5') verbunden sind, **dadurch gekennzeichnet, dass** das leitfähige Muster (101) so konfiguriert ist, dass das Ansteuern mehrerer Kombinationen der besagten Vielzahl von Verbindungsbahnen (1',2',3',4',5') mit entsprechenden Anschlüssen (1,2,3,4,5) des aufsteckbaren Datenverarbeitungsmoduls (103) zu mehreren elektrischen Schaltkreiskonfigurationen zur Messung von Widerstand, Impedanz und/oder Kapazität dadurch führt, und wobei die Vielzahl der durch das leitfähige Muster (101) definierten Schaltkreise Schaltungen mit einer offenen Konfiguration umfassen, und wobei sich das leitfähige Muster (101) symmetrisch in einer im Wesentlichen zur Länge (L) des Substrats (10) parallelen Richtung und einer im Wesentlichen dazu senkrechten Richtung erstreckt, wodurch eine Vielzahl von unabhängigen elektrischen Schaltkreisen definiert wird, und wobei Kombinationen der besagten elektrischen Schaltkreise durch selektives Ansteuern von mindestens zwei der Verbindungsbahnen (1', 2', 3', 4', 5') mit mindestens zwei entsprechenden Anschlüssen (1, 2, 3, 4, 5) des aufsteckbaren Datenverarbeitungsmoduls (103) in elektrischer Verbindung stehen, und wobei der durch die besagte Vielzahl von Schaltkreisen gemessene Widerstand, die Impedanz und/oder die Kapazität die Nässe durch entsprechende Messzonen bestimmt, die ferner direkt mit der Körperposition des Trägers zusammenhängen.

2. Das Substrat (10) nach Anspruch 1,
wobei das Substrat (10) eine zweite Oberfläche (30) aufweist, die der besagten ersten Oberfläche (20) gegenüberliegt und in der Nähe einer dem Kleidungsstück zugewandten Seite des besagten absorbierenden Artikels (100) angeordnet werden kann; und/oder
wobei das aufsteckbare Datenverarbeitungsmodul (103) in elektrischer Verbindung mit dem leitfähigen Muster (101) steht, wenn es an einer Position in der Nähe eines ersten Endes (50) des Substrats (10) angeschlossen ist, um mindestens einen geschlossenen Stromkreis zu bilden.

3. Das Substrat (10) nach Anspruch 1 oder Anspruch 2,
wobei das leitfähige Muster (101) ein elektrisch leitfähiges Material umfasst und vorzugsweise ein gedrucktes leitfähiges Muster ist,
und wobei das gedruckte leitfähige Muster (101) vorzugsweise aus einer Tinte auf Kohlenstoffbasis und/oder einer Tinte auf Basis eines leitfähigen Polymers besteht, wobei die Tinte auf Kohlenstoffbasis vorzugsweise eine leitfähige Verbindung enthält, die aus der Gruppe bestehend aus Graphen, Graphit, Nano-Kohlenstoffröhren und deren Mischungen ausgewählt ist, wobei die Tinte auf Basis eines leitfähigen Polymers vorzugsweise eine leitfähige Verbindung enthält, die aus der Gruppe bestehend aus Polyacetylen, Polypyrrol, Polyanilin und deren Copolymeren ausgewählt ist, besonders bevorzugt aus der Gruppe bestehend aus Poly(pyrrol)en (PPY), Polyanilinen (PANI), Poly(thiophen)en (PT), Poly(p-phenylensulfid) (PPS), Poly(p-phenylen) (PPP), Poly(acetylen)en (PAC), Poly(p-phenylenvinylen) (PPV), Poly(3,4-ethylendioxythiophen) (PEDOT) und deren Mischungen, wobei die am meisten bevorzugte leitfähige Tinte auf Polymerbasis Poly(3,4-ethylendioxythiophen)-Polystyrolsulfonat (PEDOT:PSS) umfasst.

4. Das Substrat (10) nach einem der vorhergehenden Ansprüche,
wobei die besagten Verbindungsbahnen (1',2',3',4',5') so konfiguriert sind, dass ein elektrischer Strom von den entsprechenden Anschlüssen (1,2,3,4,5) des aufsteckbaren Datenverarbeitungsmoduls (103) fließen kann, wenn sie damit verbunden sind, und/oder wobei sich die besagten Verbindungsbahnen (1',2',3',4',5') im Wesentlichen parallel zu einer Länge L des Substrats und im Wesentlichen parallel zu einer Längsachse (y-y) erstrecken, die ein erstes Ende (50) und ein zweites Ende (60) des Substrats (10) kreuzt; und wobei die besagten Messbahnen (9) so konfiguriert sind, dass sie bei Vorhandensein bestimmter Substanzen Veränderungen erfahren.

5. Das Substrat (10) nach einem der vorhergehenden Ansprüche,
ferner umfassend mindestens eine isolierende Schicht (200), die vorzugsweise flüssigkeitsundurchlässig ist, daran haftet und so bemessen ist, dass sie mindestens einen Teil des leitfähigen Musters (101) abdeckt, wobei die besagte mindestens eine isolierende Schicht (200) dazu ausgebildet ist, eine Abdichtung und/oder Barriere gegen das Eindringen von Flüssigkeiten und/oder Exsudaten in Kontakt mit dem besagten Teil des leitfähigen Musters (101) zu bilden, wobei vorzugsweise die Messbahnen (9) Flüssigkeiten und/oder Exsudaten ausgesetzt bleiben und nicht von der besagten mindestens einen isolierenden Schicht (200) bedeckt sind.

6. Das Substrat (10) nach einem der vorhergehenden Ansprüche,
wobei das besagte Substrat (10) einen oder mehrere Schlitze (15) aufweist,
und wobei eine Tasche (16) zwischen der ersten Oberfläche (20) und der mindestens einen isolierenden Schicht (200) in der Nähe des ersten Endes (50) gebildet wird, wobei die besagte Tasche (16) nur mit dem/den besagten Schlitz(en) (15) in Fluidverbindung steht und so angeordnet ist, dass sie mindestens einen Teil des aufsteckbaren Datenverarbeitungsmoduls (103) darin aufnimmt.

7. Das Substrat (10) nach einem der vorhergehenden Ansprüche,
wobei die Vielzahl der durch das leitfähige Muster (101) definierten Schaltkreise Schaltungen mit einer geschlossenen Konfiguration umfassen, und/oder
wobei mindestens einer der Vielzahl der durch das leitfähige Muster (101) definierten Schaltkreise eine Schaltung mit einer geschlossenen Konfiguration ist, und/oder wobei die Vielzahl der durch das leitfähige Muster (101) definierten Schaltkreise eine Konfiguration aufweisen, die das Aufbringen von Leimspuren ermöglicht, wenn sie in einen absorbierenden Artikel (100) eingearbeitet sind.

8. Das Substrat (10) nach einem der vorhergehenden Ansprüche,
wobei eine Dicke und/oder eine Länge und/oder eine Breite und/oder eine spezifische Leitfähigkeit von mindestens einem Teil von mindestens einem der Vielzahl der durch das leitfähige Muster (101) definierten Schaltkreise Kennungen des absorbierenden Artikels (100) umfassen, die erkannt werden, wenn sie mit dem aufsteckbaren Datenverarbeitungsmodul (103) verbunden sind.

9. Ein absorbierender Artikel (100), vorzugsweise eine Einwegwindel, Einlage oder Höschenwindel, der zur Überwachung und Erkennung des Vorhandenseins von Nässe darin und/oder des Risikos des Austritts von Exsudat daraus geeignet ist, wobei der besagte absorbierende Artikel umfasst:
eine flüssigkeitsundurchlässige Außenschicht (202);
eine flüssigkeitsdurchlässige Oberschicht (206);
einen absorbierenden Kern (204), der zwischen der besagten Außenschicht (202) und der Oberschicht (206) angeordnet ist, wobei die besagte Außenschicht (201) ein Substrat (10) nach einem der vorhergehenden Ansprüche umfasst.

10. Der absorbierende Artikel (100) nach Anspruch 9, wobei der besagte absorbierende Artikel ferner ein abnehmbares aufsteckbares Datenverarbeitungsmodul (103) mit einer Vielzahl von freiliegenden elektrisch leitfähigen Anschlüssen (33) aufweist, die in der Lage sind, eine Vielzahl von Verbindungsbahnen (1',2',3',4',5') eines leitfähigen Musters (101) selektiv anzusteuern, wobei vorzugsweise die Anzahl der besagten Anschlüsse (33) mindestens der Anzahl der besagten Verbindungsbahnen (1',2',3',4',5') entspricht.

11. Der absorbierende Artikel (100) nach Anspruch 10, wobei das selektive Ansteuern mehrerer Kombinationen der Vielzahl von Verbindungsbahnen (1',2',3',4',5') mit entsprechenden Anschlüssen (1,2,3,4,5) des aufsteckbaren Datenverarbeitungsmoduls (103) zu mehreren elektrischen Schaltkreiskonfigurationen zur Messung von Widerstand, Impedanz und/oder Kapazität dadurch führt; und/oder
wobei mindestens ein Teil des aufsteckbaren Datenverarbeitungsmoduls (103) in einer Tasche (16) gehalten wird, wobei die besagte Tasche über mindestens einem Teil des absorbierenden Kerns positioniert ist, wobei der Kern zwischen der besagten Tasche (16) und der Haut eines Subjekts angeordnet ist, wenn der absorbierende Artikel getragen wird, so dass, wenn das aufsteckbare Datenverarbeitungsmodul (103) in die Tasche (16) eingeführt wird, der absorbierende Kern eine Polsterschicht zwischen dem besagten aufsteckbaren Datenverarbeitungsmodul (103) und der Haut des Subjekts bereitstellt.

12. Der absorbierende Artikel (100) nach einem der Ansprüche 9 bis 11,
wobei die Messung von Widerstand, Impedanz und/oder Kapazität von mehreren Schaltkreiskonfigurationen, die durch das leitfähige Muster (101) definiert und selektiv mit entsprechenden Anschlüssen (1,2,3,4,5) des aufsteckbaren Datenverarbeitungsmoduls (103) angesteuert werden, die Überwachung der Nässe in verschiedenen Zonen des Kerns des absorbierenden Artikels (100) und/oder die Überwachung einer Verteilung der Nässe in Abhängigkeit von der aktuellen und/oder vorherigen Körperposition eines Trägers des besagten absorbierenden Artikels (100) ermöglicht.

13. Ein Verfahren zur Herstellung eines absorbierenden Artikels nach einem der Ansprüche 9 bis 12, umfassend die Schritte:
Bereitstellen einer flüssigkeitsundurchlässigen Außenschicht und Aufbringen eines leitfähigen Musters (101) nach einem der Ansprüche 1 bis 8 auf eine erste Oberfläche davon;
Bereitstellen und Anhaften mindestens einer isolierenden Schicht (200) an der besagten Außenschicht, wobei die besagte isolierende Schicht so bemessen und positioniert ist, dass sie mindestens einen Teil der Verbindungsbahnen (1',2',3',4',5') des leitfähigen Musters (101) abdeckt, um ein laminiertes Substrat bereitzustellen; Bereitstellen eines absorbierenden Kerns, der absorbierendes Material enthält;
Bereitstellen einer flüssigkeitsdurchlässigen Oberschicht;
und Sandwichartiges Anordnen des absorbierenden Kerns zwischen dem besagten laminierten Substrat und der besagten Oberschicht.

14. Das Verfahren nach Anspruch 13,
ferner umfassend den Schritt des Bereitstellens eines Schlitzes (15) und/oder einer Tasche (16) auf der Außenschicht, die es einem elektrisch leitfähigen Teil eines aufsteckbaren Datenverarbeitungsmoduls (103) ermöglicht, selektiv in elektrische Verbindung mit den Verbindungsbahnen (1',2',3',4',5') des leitfähigen Musters (101) zu treten.

15. Das Verfahren nach Anspruch 13 oder 14,
wobei das leitfähige Muster (101) in Form einer elektrisch leitfähigen Tinte vorliegt und der Aufbringungsschritt das Aufdrucken derselben auf die Außenschicht umfasst; und/oder wobei die mindestens eine isolierende Schicht (200) so geformt ist, dass sie eine Breite (w) hat, gemessen entlang einer zur Längsachse (y-y) senkrechten Achse, die geringer ist als eine Breite (W) der Außenschicht; und/oder
wobei die Überwachung und Erkennung des Vorhandenseins von Nässe in verschiedenen Zonen des Kerns des absorbierenden Artikels (100) durch Messung von Widerstand, Impedanz und/oder Kapazität von mehreren Schaltkreiskonfigurationen ermöglicht wird, die durch das leitfähige Muster (101) definiert und selektiv durch entsprechende Anschlüsse (33) des aufsteckbaren Datenverarbeitungsmoduls (103) angesteuert werden.

## Revendications

1. Un substrat (10) approprié pour être incorporé dans un article absorbant (100) pour surveiller et détecter la présence d'humidité et/ou le risque de fuite d'exsudat de celui-ci, ledit substrat (10) comprenant un motif conducteur (101) disposé sur une première surface (20) capable d'être agencé à proximité d'un côté de l'article absorbant (100) faisant face au corps, dans lequel ledit motif conducteur (101) peut être mis en communication électrique avec un module de traitement de données à clipser (103), ledit motif conducteur (101) comprenant une pluralité de pistes de connexion (1',2',3',4',5') ; et une pluralité de pistes de détection (9) connectées auxdites pistes de connexion (1',2',3',4',5'),
**caractérisé en ce que** le motif conducteur (101) est configuré de manière à ce que l'adressage de multiples combinaisons de ladite pluralité de pistes de connexion (1',2',3',4',5') avec des bornes correspondantes (1,2,3,4,5) du module de traitement de données à clipser (103) résulte en de multiples configurations de circuits électriques pour mesurer la résistance, l'impédance et/ou la capacité à travers ceux-ci, et dans lequel la pluralité de circuits définis par le motif conducteur (101) comprend des circuits avec une configuration ouverte, et dans lequel le motif conducteur (101) s'étend symétriquement dans une direction sensiblement parallèle à la longueur (L) du substrat (10) et une direction sensiblement perpendiculaire à celle-ci, définissant une pluralité de circuits électriques indépendants, et dans lequel des combinations desdits circuits électriques sont en communication électrique en adressant sélectivement au moins deux des pistes de connexion (1', 2', 3', 4', 5') avec au moins deux bornes correspondantes (1, 2, 3, 4, 5) du module de traitement de données à clipser (103), et dans lequel la résistance, l'impédance et/ou la capacité mesurées à travers ladite pluralité de circuits détermine l'humidité à travers des zones de détection correspondantes qui sont en outre directement liées à la position du corps du porteur.

2. Le substrat (10) selon la revendication 1,
dans lequel le substrat (10) comprend une seconde surface (30) opposée à ladite première surface (20) et capable d'être agencée à proximité d'un côté de l'article absorbant (100) faisant face au vêtement ; et/ou
dans lequel le module de traitement de données à clipser (103) est en communication électrique avec le motif conducteur (101) lorsqu'il est connecté à une position proximale d'une première extrémité (50) du substrat (10) de manière à former au moins un circuit électrique fermé.

3. Le substrat (10) selon la revendication 1 ou la revendication 2, dans lequel le motif conducteur (101) comprend un matériau électriquement conducteur, et est de préférence un motif conducteur imprimé,
et dans lequel le motif conducteur imprimé (101) comprend, de préférence consiste en, une encre à base de carbone et/ou une encre à base de polymère conducteur, de préférence l'encre à base de carbone comprenant un composé conducteur choisi dans le groupe constitué du graphène, du graphite, des nano-tubes de carbone et de leurs mélanges, de préférence l'encre à base de polymère conducteur comprenant un composé conducteur choisi dans le groupe constitué du polyacétylène, du polypyrrole, de la polyaniline et de leurs copolymères, plus préférentiellement choisi dans le groupe constitué des poly(pyrrole)s (PPY), des polyanilines (PANI), des poly(thiophène)s (PT), du poly(sulfure de p-phénylène) (PPS), du poly(p-phénylène) (PPP), des Poly(acétylène)s (PAC), du Poly(p-phénylène vinylène) (PPV), du poly(3,4-éthylènedioxythiophène) (PEDOT) et de leurs mélanges, l'encre à base de polymère conducteur la plus préférée comprenant du poly(3,4-éthylènedioxythiophène) polystyrène sulfonate (PEDOT:PSS).

4. Le substrat (10) selon l'une quelconque des revendications précédentes,
dans lequel lesdites pistes de connexion (1',2',3',4',5') sont configurées pour permettre à un courant électrique de circuler depuis les bornes correspondantes (1,2,3,4,5) du module de traitement de données à clipser (103) lorsqu'il y est connecté et/ou dans lequel lesdites pistes de connexion (1',2',3',4',5') s'étendent sensiblement parallèlement à une longueur L du substrat et sensiblement parallèlement à un axe longitudinal (y-y) traversant une première extrémité (50) et une seconde extrémité (60) du substrat (10) ;
et dans lequel lesdites pistes de détection (9) sont configurées pour subir des changements en présence de certaines substances.

5. Le substrat (10) selon l'une quelconque des revendications précédentes,
comprenant en outre au moins une couche isolante (200) étant de préférence imperméable aux liquides, y étant adhérée et dimensionnée pour couvrir au moins une partie du motif conducteur (101), ladite au moins une couche isolante (200) étant adaptée pour fournir un joint et/ou une barrière aux liquides et/ou exsudats pour qu'ils n'entrent pas en contact avec ladite partie du motif conducteur (101), de préférence dans lequel les pistes de détection (9) restent exposées aux liquides et/ou exsudats et ne sont pas couvertes par ladite au moins une couche isolante (200).

6. Le substrat (10) selon l'une quelconque des revendications précédentes,
dans lequel ledit substrat (10) comprend une ou plusieurs fentes (15),
et dans lequel une poche (16) est formée entre la première surface (20) et l'au moins une couche isolante (200) à proximité de la première extrémité (50), ladite poche (16) étant en communication fluidique uniquement avec ladite ou lesdites fentes (15) et agencée pour retenir au moins une partie du module de traitement de données à clipser (103) à l'intérieur.

7. Le substrat (10) selon l'une quelconque des revendications précédentes,
dans lequel la pluralité de circuits définis par le motif conducteur (101) comprend des circuits avec une configuration fermée, et/ou
dans lequel au moins un de la pluralité de circuits définis par le motif conducteur (101) est un circuit avec une configuration fermée, et/ou dans lequel la pluralité de circuits définis par le motif conducteur (101) ont une configuration qui permet d'appliquer des lignes de colle lorsqu'ils sont incorporés dans un article absorbant (100).

8. Le substrat (10) selon l'une quelconque des revendications précédentes,
dans lequel une épaisseur et/ou une longueur et/ou une largeur et/ou une conductivité spécifique d'au moins une partie d'au moins un de la pluralité de circuits définis par le motif conducteur (101) comprend des identifiants de l'article absorbant (100) qui sont détectés lorsqu'ils sont connectés au module de traitement de données à clipser (103).

9. Un article absorbant (100), de préférence une couche-culotte, une serviette ou une culotte jetable, approprié pour surveiller et détecter la présence d'humidité et/ou le risque de fuite d'exsudat de celui-ci, ledit article absorbant comprenant : une feuille de fond imperméable aux liquides (202);
une feuille de dessus perméable aux liquides (206);
un noyau absorbant (204) interposé entre ladite feuille de fond (202) et ladite feuille de dessus (206), dans lequel ladite feuille de fond (201) comprend un substrat (10) selon l'une quelconque des revendications précédentes.

10. L'article absorbant (100) selon la revendication 9, dans lequel ledit article absorbant comprend en outre un module de traitement de données à clipser amovible (103) ayant une pluralité de bornes électriquement conductrices exposées (33) capables d'adresser sélectivement une pluralité de pistes de connexion (1',2',3',4',5') d'un motif conducteur (101), de préférence dans lequel le nombre desdites bornes (33) est au moins le même que le nombre desdites pistes de connexion (1',2',3',4',5').

11. L'article absorbant (100) selon la revendication 10, dans lequel l'adressage sélectif de multiples combinaisons de la pluralité de pistes de connexion (1',2',3',4',5') avec des bornes correspondantes (1,2,3,4,5) du module de traitement de données à clipser (103) résulte en de multiples configurations de circuits électriques pour mesurer la résistance, l'impédance et/ou la capacité à travers ceux-ci ; et/ou
dans lequel au moins une partie du module de traitement de données à clipser (103) est retenue à l'intérieur d'une poche (16), ladite poche étant positionnée sur au moins une partie du noyau absorbant, le noyau étant interposé entre ladite poche (16) et la peau d'un sujet lorsque l'article absorbant est porté, de sorte que lorsque le module de traitement de données à clipser (103) est inséré dans la poche (16), le noyau absorbant fournit une couche de rembourrage entre ledit module de traitement de données à clipser (103) et ladite peau du sujet.

12. L'article absorbant (100) selon l'une quelconque des revendications 9 à 11,
dans lequel la mesure de la résistance, de l'impédance et/ou de la capacité de multiples configurations de circuits définies par le motif conducteur (101) adressées sélectivement avec des bornes correspondantes (1,2,3,4,5) du module de traitement de données à clipser (103) permet de surveiller l'humidité dans différentes zones du noyau de l'article absorbant (100) et/ou de surveiller une distribution de l'humidité en fonction de la position corporelle actuelle et/ou antérieure d'un porteur dudit article absorbant (100).

13. Un procédé de fabrication d'un article absorbant selon l'une quelconque des revendications 9 à 12, comprenant les étapes de:
fournir une feuille de fond imperméable aux liquides et appliquer un motif conducteur (101), selon l'une quelconque des revendications 1 à 8, sur une première surface de celle-ci;
fournir et adhérer au moins une couche isolante (200) à ladite feuille de fond, ladite couche isolante étant dimensionnée et positionnée pour couvrir au moins une partie des pistes de connexion (1',2',3',4',5') du motif conducteur (101), pour fournir un substrat laminé;
fournir un noyau absorbant comprenant un matériau absorbant;
fournir une feuille de dessus perméable aux liquides; et prendre en sandwich le noyau absorbant entre ledit substrat laminé et ladite feuille de dessus.

14. Le procédé selon la revendication 13, comprenant en outre l'étape de fournir une fente (15) et/ou une poche (16) sur la feuille de fond permettant à une partie électriquement conductrice d'un module de traitement de données à clipser (103) d'entrer sélectivement en communication électrique avec les pistes de connexion (1',2',3',4',5') du motif conducteur (101).

15. Le procédé selon les revendications 13 ou 14, dans lequel le motif conducteur (101) est sous la forme d'une encre électriquement conductrice et l'étape d'application comprend son impression sur la feuille de fond ; et/ou dans lequel l'au moins une couche isolante (200) est formée pour avoir une largeur (w), prise le long d'un axe perpendiculaire à l'axe longitudinal (y-y), étant inférieure à une largeur (W) de la feuille de fond; et/ou dans lequel la surveillance et la détection de la présence d'humidité dans différentes zones du noyau de l'article absorbant (100) sont permises par la mesure de la résistance, de l'impédance et/ou de la capacité de multiples configurations de circuits définies par le motif conducteur (101) et adressées sélectivement par des bornes correspondantes (33) du module de traitement de données à clipser (103).
